# EUROPEAN PATENT SPECIFICATION

(11) **EP 1 866 310 B1**
(45) Date of publication and mention of the grant of the patent: **11.07.2012**
(21) Application number: 06710177.4
(22) Date of filing: 21.03.2006
(51) Int. Cl.: C07D 473/32, C07D 471/04, C07D 495/04, C07D 513/04, A61K 31/522, A61P 29/00

(54) **2,3-SUBSTITUTED FUSED PYRIMIDIN-4(3H)-ONES AS VR1 ANTAGONISTS**
2,3-SUBSTITUIERTE KONDENSIERTE PYRIMIDIN-4(3H)-ONE ALS VR1-ANTAGONISTEN
PYRIMIDINE-4(3H)-ONES FONDUES SUBSTITUEES EN 2,3 COMME ANTAGONISTES VR1

(30) Priority: 24.03.2005 GB 0506147
(43) Date of publication of application: 19.12.2007
(73) Proprietor: MERCK SHARP & DOHME LTD., Hoddesdon, Hertfordshire EN11 9BU (GB)
(72) Inventor: BAYLISS, Tracy, Harlow, Essex CM20 2QR (GB); BROWN, Rebecca Elizabeth, Harlow, Essex CM20 2QR (GB); HOLLINGWORTH, Gregory John, Harlow, Essex CM20 2QR (GB); JONES, A. Brian, Harlow, Essex CM20 2QR (GB); MOYES, Christopher Richard, Harlow, Essex CM20 2QR (GB); ROGERS, Lauren, Harlow, Essex CM20 2QR (GB)
(74) Representative: Horgan, James Michael Frederic
(86) International application number: PCT/GB2006/050060
(87) International publication number: WO 2006/100520

(56) References cited:
- WO-A-02/076946
- WO-A-03/014064
- WO-A-2005/049613
- US-A- 3 674 786
- DATABASE CAPLUS CHEMICAL ABSTRACTS SERVICE, COLUMBUS, OHIO, US; XP002385043 retrieved from STN accession no. 1990:178855 Database accession no. 112:178855 & PHOSPHORUS, SULFUR AND SILICON AND THE RELATED ELEMENTS, vol. 44, no. 3-4, 1989, pages 197-201,
- PATHAK U ET AL: "SYNTHESIS AND BIOLOGICAL ACTIVITIES OF SOME 2-(NNDISUBSTIT.AMINO)3-PH ENYLTHIENOPYRIMIDINE-4-ONES" CHEMICAL ABSTRACTS + INDEXES, AMERICAN CHEMICAL SOCIETY. COLUMBUS, US, vol. 28, no. 115, 1991, page 919, XP002033221 ISSN: 0009-2258
- DATABASE BEILSTEIN BEILSTEIN INSTITUTE FOR ORGANIC CHEMISTRY, FRANKFURT-MAIN, DE; XP002385044 Database accession no. BRN:4820078 & INDIAN J. CHEM. SECT. B, vol. 30, no. 6, 1991, pages 618-619,
- DATABASE BEILSTEIN BEILSTEIN INSTITUTE FOR ORGANIC CHEMISTRY, FRANKFURT-MAIN, DE; XP002385045 Database accession no. BRN:6756214 & J. INDIAN CHEM. SOC., vol. 66, 1989, pages 810-812,
- DATABASE BEILSTEIN BEILSTEIN INSTITUTE FOR ORGANIC CHEMISTRY, FRANKFURT-MAIN, DE; XP002385046 Database accession no. BRN:692384 & SYNTH. COMMUN., vol. 30, no. 9, 2000, pages 1599-1604,

## Description

The present invention is concerned with 2,3-substituted fused pyrimidin-4(3H)-ones and analogues and derivatives thereof as well as pharmaceutically acceptable salts and prodrugs thereof, which are useful as therapeutic compounds, particularly in the treatment of pain and other conditions ameliorated by the modulation of the function of the vanilloid-1 receptor (VR1, also known as TRPV1).

The pharmacologically active ingredient of chilli peppers has been recognised for some time to be the phenolic amide capsaicin. The application of capsaicin to mucous membranes or when injected intradermally, causes intense burning-like pain in humans. The beneficial effects of topical administration of capsaicin as an analgesic is also well established. However, understanding of the underlying molecular pharmacology mediating these responses to capsaicin has been a more recent development.

The receptor for capsaicin, termed the vanilloid VR1 receptor, was cloned by Caterina and colleagues at UCSF in 1997 (Nature, 398:816, 1997). VR1 receptors are cation channels that are found on sensory nerves that innervate the skin, viscera, peripheral tissues and spinal cord. Activation of VR1 elicits action potentials in sensory fibres that ultimately generate the sensation of pain. Importantly the VR1 receptor is activated not only by capsaicin but also by acidic pH and by noxious heat stimuli. It is also sensitized by a number of inflammatory mediators and thus appears to be a polymodal integrator of painful stimuli.

The prototypical VR1 antagonist is capsazepine (Walpole et al., J. Med. Chem., 37:1942, 1994) - VR1 IC₅₀ of 420nM. Other sub-micromolar antagonists has also been reported recently (Lee et al, Bioorg. Med. Chem., 9:1713, 2001; Park et al, Bioorg. Med. Chem. Lett., 13:601, 2003; Yoon et al, Bioorg. Med. Chem. Lett., 13:1549, 2003; Lee et al, Bioorg. Med. Chem., 12:3411, 2004; McDonnell et al, Bioorg. Med. Chem. Lett., 14:531, 2004; Ryu et al, Bioorg. Med. Chem. Lett., 14:1751, 2004; Rami et al, Bioorg. Med. Chem. Lett., 14:3631, 2004; Gunthorpe et al, Neuropharmacology 46:133, 2004; Doherty et al, J. Med. Chem., 48:71, 2005), but these reports provide no evidence for *in vivo* efficacy. A high affinity antagonist has been derived from the potent agonist resiniferatoxin; iodo-resiniferatoxin (Wahl et al., Mol. Pharmacol., 59:9, 2001) is a nanomolar antagonist of VR1 but does not possess properties suitable for an oral pharmaceutical. This last is also true of the micromolar peptoid antagonists described by Garcia-Martinez (Proc. Natl. Acad Sci., USA, 99:2374, 2002).

EP-A-0807633 (Pfizer Inc.) discloses structurally related AMPA receptor antagonists for treating neurodegenerative and GNS-trauma related conditions.

WO-A-9733890 (Novartis AG) discloses structurally related compounds as pesticides.

The compounds of the present invention have advantageous properties, such as good *in vivo* efficacy.

We herein describe another novel series of VR1 modulators. These comprise predominantly VR1 antagonists but encompass VR1 partial antagonists and VR1 partial agonists. Such compounds have been shown to be efficacious in animal models of pain.

The present invention provides compounds of formula I: 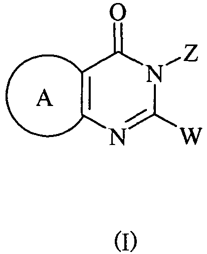 wherein:
Wis 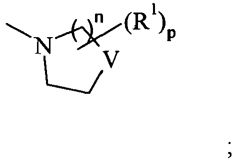
A is a five-membered heteroaromatic ring containing 2 N heteroatoms;
A is optionally substituted by one, two or three groups independently chosen from halogen, hydroxy, S(O)ᵣC₁₋₆alkyl, S(O)ᵣNR²R³, formyl, C₁₋₆alkylcarbonyl, C₁₋₆alkyl, haloC₁₋₄alkyl, hydroxyC₁₋₆alkyl, C₁₋₆alkoxy, haloC₁₋₆alkoxy, hydroxyC₁₋₆alkoxy, C₃-₇cycloalkyl, C₃₋₇cycloalkoxy, C₂₋₆alkenyl, C₂₋₆alkynyl, amino, nitro, cyano, C₁₋₆alkylamino, di(C₁₋₆alkyl)amino, aminoC₁₋₆alkyl, aminoC₁₋₆alkoxy, C₁₋₆alkylaminoC₁₋₆alkyl, di(C₁₋₆alkyl)aminoC₁₋₆alkyl; and a ring selected from phenyl, naphthyl, a five-membered heteroaromatic ring containing one, two, three or four heteroatoms independently chosen from O, N or S, at most one heteroatom being O or S, and a six-membered heteroaromatic ring containing one, two or three N atoms, the ring being optionally substituted by halogen, hydroxy, cyano, nitro, NR²R³ as defined below, C₁₋₆alkyl, C₂₋₆alkenyl, C₂₋₆alkynyl, haloC₁₋₆alkyl, C₁₋₆alkoxy, haloC₁₋₆alkoxy, C₃₋₇cycloalkyl or hydroxyC₁₋₆alkyl;

   R¹ is X-Y-R⁴;
each R² and R³ is independently hydrogen or C₁₋₆alkyl or R² and R³, together with the nitrogen atom to which they are attached, may form a saturated 4-7 membered ring;
n is zero, one, two or three;
when n is zero or one, V is CH₂;
when n is two or three, V is CH₂, O or NR⁵;
when V is CH₂, the bond formed by V and an adjacent carbon ring atom is optionally fused to a phenyl ring, a five-membered heteroaromatic ring containing 1, 2 or 3 heteroatoms independently chosen from O, N and S, providing that no more than one O or S is present, or a six-membered heteroaromatic ring containing 1, 2 or 3 N atoms; the ring being optionally substituted by one or more R¹ groups;
R⁵ is hydrogen or together with an adjacent N-C ring bond forms a fused five-membered heteroaromatic ring containing one, two, three or four nitrogen atoms optionally substituted by one or more R¹ groups;
X is a bond, O or NR⁶;
Y is (CR⁷R⁸)ₐ;
each R⁴ is independently halogen, hydroxy, cyano, C₁₋₆alkyl, haloC₁₋₆alkyl, hydroxyC₁₋₆alkyl, C₁₋₆alkoxy, haloC₁₋₆alkoxy, hydroxyC₁₋₆alkoxy, C₃₋₇cycloalkyl, C₃₋₇cycloalkylC₁₋₆alkyl, formyl, C₁₋₆alkylcarbonyl, carboxy, NR²R³, CONR²R³, S(O)ᵣNR²R³; or a ring which is phenyl; naphthyl; a five-membered heteroaromatic ring containing one, two, three or four heteroatoms independently chosen from O, N and S, at most one heteroatom being O or S; a six-membered heteroaromatic ring containing one, two or three N atoms; or a six-membered saturated ring containing one or two heteroatoms independently chosen from O and N; the ring being optionally substituted by one or more groups independently selected from halogen, C₁₋₆alkyl, C₂₋₆alkenyl, C₂₋₆alkynyl, nitro, cyano, C₃₋₇cycloalkyl, hydroxy, C₁₋₆alkoxy haloC₁₋₆alkyl, haloC₁₋₆alkoxy, hydroxyC₁₋₆alkyl, hydroxyC₁₋₆alkoxy and NR²R³;
R⁶ is hydrogen or C₁₋₄alkyl;
R⁷ and R⁸ are independently hydrogen, hydroxy, halogen or C₁₋₄alkyl;
Z is a phenyl ring, a five-membered heteroaromatic ring containing one, two, three or four heteroatoms independently chosen from O, N or S, at most one heteroatom being O or S, or a six-membered heteroaromatic ring containing one, two or three N atoms, optionally substituted by one or more groups chosen from halogen, hydroxy, cyano, nitro, NR²R³ or S(O)ᵣNR²R³ where NR²R³ is as defined above, C₁₋₆alkyl, C₂₋₆alkenyl, C₂₋₆alkynyl, haloC₁₋₆alkyl, C₁₋₆alkoxy, haloC₁₋₆alkoxy, C₁₋₆alkylthio, haloC₁₋₆alkylthio,C₃₋₇cycloalkyl, hydroxyC₁₋₆alkyl, a five-membered heteroaromatic ring containing 1, 2 or 3 heteroatoms independently chosen from O, N and S, providing that no more than one O or S atom is present, or a six-membered heteroaromatic ring containing 1, 2 or 3 N atoms;
a is zero, one, two, three or four;
p is zero, one, two or three;
r is one or two;
provided that when p is zero then R⁵ is not H; and when p is one or two and R⁵ is H then at least one group R¹ is other than halogen, hydroxy and C₁₋₄alkyl;
or a pharmaceutically acceptable salt or N-oxide thereof.

In one embodiment:
W is

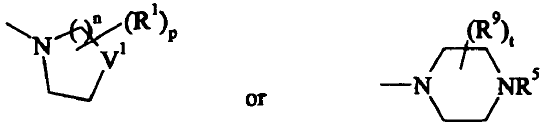

wherein R¹ is as defined above;
p is zero, one, two or three;
n is zero, one, two or three;
when n is zero or one, V¹ is CH₂;
when n is two or three, V¹ is CH₂ or O;
when V¹ is CH₂, the bond formed by V¹ and an adjacent carbon ring atom is optionally fused to a phenyl ring, a five membered heteroaromatic ring containing 1, 2 or 3 heteroatoms independently chosen from O, N and S, providing that no more than one O or S is present, or a six membered heteroaromatic ring containing 1, 2 or 3 N atoms; the ring being optionally substituted by one or more R¹ groups;
R⁵ is hydrogen or together with an adjacent N-C ring bond forms a fused five-membered heteroaromatic ring containing one, two, three or four nitrogen atoms optionally substituted by one or more R¹ groups;
when R⁵ is hydrogen, t is one, two or three;
when R⁵ together with an adjacent N-C ring bond forms a fused ring, t is zero, one, two or three; and
each R⁹ is independently cyano, haloC₁₋₆alkyl, hydroxyC₁₋₆alkyl, C₁₋₆alkoxy, haloC₁₋₆alkoxy, hydroxyC₁₋₆alkoxy, C₃₋₇cycloalkyl, C₃₋₇cycloalkylC₁₋₆alkyl, formyl, C₁₋₆alkylcarbonyl, carboxy, NR²R³, CONR²R³, S(O)ᵣNR²R³; or a ring which is phenyl; naphthyl; a five-membered heteroaromatic ring containing one, two, three or four heteroatoms independently chosen from O, N and S, at most one heteroatom being O or S; a six-membered heteroaromatic ring containing one, two or three N atoms; or a six-membered saturated ring containing one or two heteroatoms independently chosen from O and N, the ring being optionally substituted by one or more groups independently selected from halogen, C₁₋₆alkyl, C₂₋₆alkenyl, C₂₋₆alkynyl, nitro, cyano, C₃₋₇cycloalkyl, hydroxy, C₁₋₆alkoxy, haloC₁₋₆alkyl, haloC₁₋₆alkoxy, hydroxyC₁₋₆alkyl, hydroxyC₁₋₆alkoxy and NR²R³.

In another embodiment:
W is 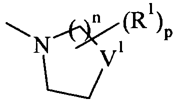 wherein n, p, R¹ and V¹ are as defined above.

A is preferably an optionally substituted five-membered heteroaromatic ring containing 2 N heteroatoms.

More particularly A is an optionally substituted imidazole ring.

A is preferably optionally substituted by one, two or three groups independently chosen from halogen, hydroxy, C₃₋₆cycloalkyl, C₁₋₄alkyl, haloC₁₋₄alkyl, C₁₋₄alkoxy, haloC₁₋₄alkoxy, phenyl, hydroxyC₁₋₄alkyl, aminoC₁₋₄alkyl, C₁₋₄alkylaminoC₁₋₄alkyl and di(C₁₋₄alkyl)aminoC₁₋₄alkyl. A particularly favoured substituent is C₁₋₄alkyl.

Favourably the optional substituents on A are chosen from methyl, ethyl, propyl, isopropyl, hydroxyethyl, cyclopropyl, cyclopropylmethyl, phenyl or dimethylaminoethyl. Particularly favoured substituents are methyl and ethyl.

A is preferably unsubstituted or substituted by one or two groups. More particularly A is unsubstituted or monosubstituted.

Thus, A is preferably an imidazole ring unsubstituted or monosubstituted by methyl or ethyl.

When A is substituted by hydroxy group tautomerism may occur. For example when A is fused imidazole, tautomerism may occur to form an imidazolone.

X is preferably a bond.

Y is preferably (CH₂)ₐ wherein a is zero or one.

Preferably, when n is two or three, V is CH₂ or O.

Preferably, n is zero, one or two.

Preferably, when V or V¹ is CH₂, the bond formed by V or V¹ and an adjacent carbon ring atom is optionally fused to a ring selected from phenyl, pyridine, pyrimidine, thiophene and thiazole, the ring being optionally substituted by halogen or haloC₁₋₆alkyl. More particularly the optional substituent on the fused ring is fluorine, chlorine or trifluoromethyl.

Preferably, the fused ring is unsubstituted or substituted by one or two groups. More particularly, the fused ring is unsubstituted or monosubstituted.

R⁴ is preferably halogen, C₁₋₆alkyl, haloC₁₋₆alkyl, C₁₋₆alkoxy, a ring which is phenyl or a five-membered heteroaromatic ring containing one, two, three or four heteroatoms independently chosen from O, N and S, at most one heteroatom being O or S, the ring being optionally substituted by C₁₋₆alkyl. More preferably R⁴ is fluorine, methyl, trifluoromethyl, methoxy, phenyl or an optionally substituted ring selected from oxadiazole and triazole, the optional substituent being selected from methyl and ethyl.

Particular R⁴ groups are fluorine, methyl, chlorine, trifluoromethyl, methoxy, phenyl, 3-ethyl[1,2,4]oxadiazol-5-yl and 4-methyl-4H-[1,2,4]triazo-3-yl.

Preferably, R⁵ together with an adjacent N-C ring bond forms a fused five-membered heteroaromatic ring containing one, two, three or four nitrogen atoms optionally substituted by one or more R¹ groups.

Preferably, the fused five-membered heteroaromatic ring is unsubstituted or substituted by one, two or three groups independently selected from R¹. More particularly, the fused five-membered heteroaromatic ring is unsubstituted, monosubstituted or disubstituted. Preferably, the fused five-membered heteroaromatic ring is monosubstituted.

Specifically, R⁵ together with an adjacent N-C ring bond forms a fused imidazole ring optionally substituted by haloC₁₋₄alkyl, particularly trifluoromethyl.

R⁶ is preferably hydrogen or methyl.

R⁷ and R⁸ are independently preferably hydrogen, methyl or ethyl. Favourably R⁷ and R⁸ are both hydrogen.

R⁹ is preferably haloC₁₋₆alkyl, C₁₋₆alkoxy, a ring which is phenyl or a five-membered heteroaromatic ring containing one, two, three or four heteroatoms independently chosen from O, N and S, at most one heteroatom being O or S, the ring being optionally substituted by C₁₋₄alkyl. More preferably R⁹ is trifluoromethyl, methoxy, phenyl or an optionally substituted ring selected from oxadiazole and triazole, the optional substituent being selected from methyl and ethyl.

a is preferably zero or one. In one embodiment a is zero.

Thus, particular W groups are piperidin-1-yl, 2-methylpyrrolidin-1-yl, pyrrolidin-1-yl, morpholin-4-yl, 4-trifluornmethylpiperidin-1-yl, 3-trifluoromethylpiperidin-1-yl, 3-methylpiperidin-1-yl, 3,3-dimethylpiperidin-1-yl, 4,4-difluoropiperidin-1-yl, 3-methoxypiperidin-1-yl, 3-benzylpiperidin-1-yl, 3-phenyl-1-piperidinyl, 3,3,difluoropiperidin-1-yl, 3-trifluoromethylpyrrolidin-1-yl, 3-(trifluoromethyl)-7,8-dihydro-1,6-naphthyridin-6(5*H*)-yl, azetidin-1-yl, 2-(trifluoromethyl)-5,8-dihydropyrido[3,4-*d*]pyrimidin-7(6*H*)-yl, 2-(trifluoromethyl)-6,7-dihydro[1,3]thiazolo[4,5-*c*]pyridin-5(4*H*)-yl, 2-(trifluoromethyl)-5,8-dihydro-1,7-naphthyridin-7(6*H*)-yl, 3-(4-methyl-4*H*-1,2,4-triazol-3-yl)piperidin-1-yl, 3-(3-ethyl-1,2,4-oxadiazol-5-yl)-piperidin-1-yl, 3,4-dihydroisoquinolin-2(1*H*)-yl, 6,7-dihydrothieno[3,2-*c*]pyridin-5(4*H*)-yl, 3-phenylpyrrolidin-1-yl, 7-fluoro-3,4-dihydroisoquinolin-2(1*H*)-yl, 7-chloro-3,4-dihydroisoquinolin-2(1*H*)-yl and 2-(trifluoromethyl)-5,6-dihydroimidazo[1,2-*a*]pyrazin-7(8*H*)-yl.

Z is preferably an optionally substituted phenyl or pyridinyl. More preferably Z is an optionally substituted phenyl.

Z is preferably unsubstituted or substituted by one or two groups. More particularly Z is monosubstituted or disubstituted.

Z is preferably unsubstituted or substituted by one or two substituents independently chosen from cyano, halogen, C₁₋₄alkyl, haloC₁₋₄alkyl, C₁₋₄alkoxy, haloC₁₋₄alkoxy, amino, C₁₋₆alkylamino and di(C₁₋₄alkyl)amino. More particularly Z is substituted by one or two groups independently chosen from chlorine, fluorine and cyano.

Thus, particularly preferred Z groups are 4-chlorophenyl, 4-fluorophenyl, 4-cyanophenyl and 3,4-difluorophenyl.

Preferably p is zero, one or two.

The present invention also provides compounds of formula IA: 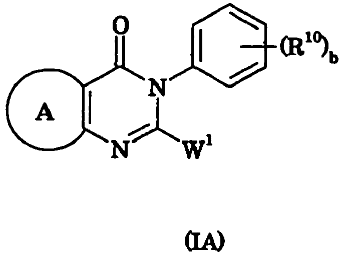 wherein:
b is zero, one, two or three;
A is as defined above;
R¹⁰ is halogen or cyano;
W¹ is: 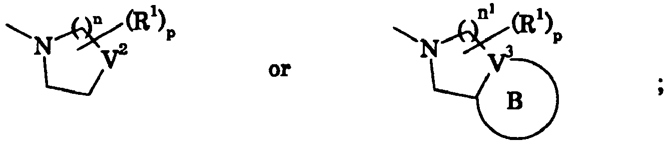
R¹ is as defined above;
p is zero, one or two;
n is zero, one or two;
n¹ is two or three;
when n is zero or one, V² is CH₂;
when n is two, V² is CH₂ or O;
V³ is C or N;
when V³ is C, B is a phenyl ring, a five-membered heteroaromatic ring containing 1, 2 or 3 heteroatoms independently chosen from O, N and S, providing that no more than one O or S is present , or a six-membered heteroaromatic ring containing 1, 2 or 3 N atoms;
when V³ is N, B is an imidazole ring;
B is optionally substituted by one, two or three groups selected from halogen, C₁₋₆alkyl, haloC₁₋₆alkyl and C₁₋₆alkoxy;
or a pharmaceutically acceptable salt or N-oxide thereof.

The preferred identities with reference to formula IA are as defined previously *mutatis mutandis.* In one embodiment W¹ is: 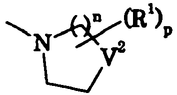 wherein n, p, R¹ and V² are as defined above.

In another embodiment W¹ is: 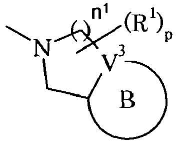 wherein n¹, p, B, R¹ and V³ are as defined above.

b is preferably zero, one or two. More particularly b is one or two.

R¹⁰ is preferably halogen or cyano. More preferably R¹⁰ is fluorine, chlorine or cyano.

A is preferably a fused imidazole ring unsubstituted or monosubstituted by C₁₋₄alkyl, especially methyl or ethyl.

Preferably, n¹ is two.

Preferably, the optional substituents on B are selected from halogen and haloC₁₋₆alkyl. More particularly, the optional substituents on B are selected from fluorine, chlorine and trifluoromethyl.

Preferably, B is unsubstituted or substituted by one or two groups. More particularly, B is unsubstituted or monosubstituted.

Preferably, when V³ is C, B is an optionally substituted ring selected from phenyl, pyridine, pyrimidine, thiophene or thiazole.

More particularly, when V³ is C, B is phenyl, pyridine, pyrimidine, thiophene or thiazole optionally monosubstituted by halogen or haloC₁₋₆alkyl, especially fluorine, chlorine or trifluoromethyl.

Preferably, when V³ is N, B is an imidazole ring optionally substituted by haloC₁₋₆alkyl, especially trifluoromethyl.

The present invention also provides compounds of formula IB: 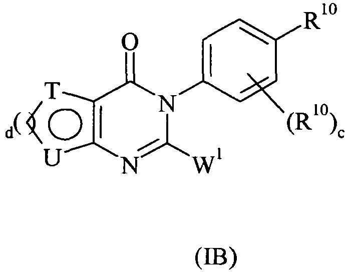 wherein
R¹⁰ and W¹ are as defined with reference to formula IA;
c is zero or one;
d is one or two;
TisN;
when T is N, U is N;
or a pharmaceutically acceptable salt or N-oxide thereof.

The preferred identities with reference to formula IB are as defined previously *mutatis mutandis.* In one embodiment, c is zero.

Particular compounds of the invention include:
1-(4-chlorophenyl)-9-methyl-2-piperidin-1-yl-1,9-dihydro-6*H*-purin-6-one;
1-(4-chlorophenyl)-9-methyl-2-(2-methylpyrrolidin-1-yl)-1,9-dihydro-6*H*-purin-6-one hydrochloride;
1-(4-chlorophenyl)-9-methyl-2-pyrrolidin-1-yl-1,9-dihydro-6*H*-purin-6-one;
1-(4-chlorophenyl)-9-methyl-2-morpholin-4-yl-1,9-dihydro-6*H*-purin-6-one;
1-(4-chlorophenyl)-9-methyl-2-[4-(trifluoromethyl)piperidin-1-yl]-1,9-dihydro-6*H*-purin-6-one;
1-(4-chlorophenyl)-9-methyl-2-[3-(trifluoromethyl)piperidin-1-yl]-1,9-dihydro-6*H*-purin-6-one;
1-(4-chlorophenyl)-9-ethyl-2-(3-methylpiperidin-1-yl)-1,9-dihydro-6*H*-purin-6-one;
1-(4-chlorophenyl)-2-(3,3-dimethylpiperidin-1-yl)-9-ethyl-1,9-dihydro-6*H*-purin-6-one;
1-(4-chlorophenyl)-2-(4,4-difluoropiperidin-1-yl)-9-ethyl-1,9-dihydro-6*H*-purin-6-one;
1-(4-chlorophenyl)-9-ethyl-2-(3 -methoxypiperidin-1-yl)-1,9-dihydro-6*H*-purin-6-one;
2-(3-benzylpiperidin-1-yl)-1-(4-chlorophenyl)-9-ethyl-1,9-dihydro-6*H*-purin-6-one;
1-(4-chlorophenyl)-9-ethyl-1,9-dihydro-2-(3-phenyl-1-piperidinyl)-6*H*-purin-6-one;
1-(4-chlorophenyl)-2-(3,3-difluoropiperidin-1-yl)-9-ethyl-1,9-dihydro-6*H*-purin-6-one;
1-(4-chlorophenyl)-9-ethyl-2-[3-(trifluoromethyl)pyrrolidin-1-yl]-1,9-dihydro-6*H*-purin-6-one;
1-(4-chlorophenyl)-9-ethyl-2-[3-(trifluoromethyl)piperidin-1-yl]-1,9-dihydro-6*H*-purin-6-one;
2-azetidin-1-yl-1-(4-chlorophenyl)-9-ethyl-1,9-dihydro-6*H*-purin-6-one;
1-(4-chlorophenyl)-9-methyl-2-[2-(trifluoromethyl)-5,8-dihydropyrido[3,4-*c*]pyrimidin-7(6*H*)-yl]-1,9-dihydro-6*H*-purin-6-one;
1-(4-chlorophenyl)-9-ethyl-2-[2-(trifluoromethyl)-6,7-dihydro[1,3]thiazolo[4,5-*c*]pyridin-5(4*H*)-yl]-1,9-dihydro-6*H*-purin-6-one;
4-{9-methyl-6-oxo-2-[3-(trifluoromethyl)piperidin-1-yl]-6,9-dihydro-1*H*-purin-1-yl}benzonitrile;
1-(4-chlorophenyl)-9-methyl-2-[2-(trifluoromethyl)-5,8-dihydro-1,7-naphthyridin-7(6*H*)-yl]-1,9-dihydro-6*H*-purin-6-one;
1-(3,4-difluorophenyl)-9-methyl-2-[3-(trifluoromethyl)piperidin-1-yl]-1,9-dihydro-6*H*-purin-6-one;
1-(4-chlorophenyl)-9-methyl-2-[3-(trifluoromethyl)-7,8-dihydro-1,6-naphthyridin-6(5*H*)-yl]-1,9-dihydro-6*H*-purin-6-one;
1-(4-fluorophenyl)-9-methyl-2-[3-(trifluoromethyl)piperidin-1-yl]-1,9-dihydro-6*H*-purin-6-one;
1-(4-chlorophenyl)-9-ethyl-2-[3-(trifluoromethyl)-7,8-dihydro-1,6-naphthyridin-6(5*H*)-yl]-1,9-dihydro-6*H*-purin-6-one;
1-(4-chlorophenyl)-9-ethyl-2-[3-(4-methyl-4*H*-1,2,4-triazol-3-yl)piperidin-1-yl]-1,9-dihydro-6*H*-purin-6-one;
1-(4-chlorophenyl)-9-ethyl-2-[3-(3-ethyl-1,2,4-oxadiazol-5-yl)piperidin-1-yl]-1,9-dihydro-6*H*-purin-6-one;
1-(4-chlorophenyl)-2-(3,4-dihydroisoquinolin-2(1*H*)-yl)-9-ethyl-1,9-dihydro-6*H*-purin-6-one;
1-(4-chlorophenyl)-2-(6,7-dihydrothieno[3,2-*c*]pyridin-5(4*H*)-yl)-9-ethyl-1,9-dihydro-6*H*-purin-6-one;
1-(4-chlorophenyl)-9-methyl-2-(3-phenylpyrrolidin-1-yl)-1,9-dihydro-6*H*-purin-6-one;
1-(4-chlorophenyl)-9-ethyl-2-(3-phenylpyrrolidin-1-yl)-1,9-dihydro-6*H*-purin-6-one;
1-(4-chlorophenyl)-2-(7-fluoro-3,4-dihydroisoquinolin-2(1*H*)-yl)-9-methyl-1,9-dihydro-6*H*-purin-6-one;
2-(7-chloro-3,4-dihydroisoquinolin-2(1*H*)-yl)-1-(4-chlorophenyl)-9-methyl-1,9-dihydro-6*H*-purin-6-one;
2-(7-fluoro-3,4-dihydroisoquinolin-2(1*H*)-yl)-1-(4-fluorophenyl)-9-methyl-1,9-dihydro-6*H*-purin-6-one;
2-(7-chloro-3,4-dihydroisoquinolin-2(1*H*)-yl)-1-(4-fluorophenyl)-9-methyl-1,9-dihydro-6*H*-purin-6-one; and
1-(4-chlorophenyl)-9-ethyl-2-[2-(trifluoromethyl)-5,6-dihydroimidazo[1,2-a]pyrazin-7(8*H*)-yl]-1,9-dihydro-6*H*-purin-6-one,
or a pharmaceutically acceptable salt or N-oxide thereof.

For the avoidance of doubt, the R¹ group may be substituted at any substitutable ring position of W. It will be clear to a person skilled in the art that when V or V¹ represents CH₂, and the bond formed by V or V¹ and an adjacent carbon ring atom is fused to an aromatic ring then V or V¹ is C.

When any variable (e.g. R¹ and R², etc.) occurs more than one time in any constituent, its definition on each occurrence is independent at every other occurrence. Also, combinations of substituents and variables are permissible only if such combinations result in stable compounds. Lines drawn into the ring systems from substituents represent that the indicated bond may be attached to any of the substitutable ring atoms.

It is understood that substituents and substitution patterns on the compounds of the instant invention can be selected by one of ordinary skill in the art to provide compounds that are chemically stable and that can be readily synthesized by techniques known in the art, as well as those methods set forth below, from readily available starting materials. If a substituent is itself substituted with more than one group, it is understood that these multiple groups may be on the same carbon or on different carbons, so long as a stable structure results. The phrase "optionally substituted" should be taken to be equivalent to the phrase "unsubstituted or substituted with one or more substituents" and in such cases the preferred embodiment will have from zero to three substituents. More particularly, there are zero to two substituents. A substituent on a saturated, partially saturated or unsaturated heterocycle can be attached at any substitutable position.

As used herein, the term "alkyl" or "alkoxy" as a group or part of a group means that the group is straight or branched. Examples of suitable alkyl groups include methyl, ethyl, n-propyl, i-propyl, n-butyl, s-butyl and t-butyl, and most especially methyl and ethyl. Examples of suitable alkoxy groups include methoxy, ethoxy, n-propoxy, i-propoxy, n-butoxy, s-butoxy and t-butoxy, and most especially methoxy.

As used herein, the term 'C₁₋₆alkylthio' means a C₁₋₆alkyl radical attached via a S atom. Suitable examples are methylthio and ethylthio.

As used herein, the terms "haloC₁₋₆alkyl", "haloC₁₋₆alkoxy" and "haloC₁₋₆alkylthio" mean a C₁₋₆alkyl, C₁₋₆alkoxy or C₁₋₆alkylthio group in which one or more (in particular, 1 to 3) hydrogen atoms have been replaced by halogen atoms, especially fluorine or chlorine atoms. Preferred are fluoroC₁₋₆alkyl, fluoroC₁₋₆alkoxy and fluoroC₁₋₆alkylthio groups, in particular, fluoroC₁₋₃alkyl, fluoroC₁₋₃alkoxy and fluoroC₁₋₃alkylthio groups, for example, CF₃, CH₂F, CHF₂, CH₂CH₂F, CH₂CHF₂, CH₂CF₃, OCF₃, OCH₂CH₂F, OCH₂CHF₂, OCH₂CF₃, SCF₃, SCH₂CH₂F, SCH₂CHF₂, SCH₂CF₃ and most especially CF₃, OCF₃ and SCF₃.

The terms 'hydroxyC₁₋₆alkyl' and 'hydroxyC₁₋₆alkoxy' shall be construed in an analogous manner. Particularly preferred are hydroxyC₁₋₃alkyl and hydroxyC₁₋₈alkoxy groups, for example, CH₂OH, CH₂H₂OH, CH(CH₃)OH, C(CH₃)₂OH, OCH₂OH, OCH₂H₂OH, OCH(CH₃)OH, OC(CH₃)₂OH, and most especially CH₂OH and OCH₂OH.

As used herein, the term "C₁₋₄alkylcarbonyl" denotes a C₁₋₆alkyl radical attached via a carbonyl (C=O) radical. Suitable examples are methylcarbonyl, ethylcarbonyl and propylcarbonyl.

The cycloalkyl groups referred to herein may represent, for example, cyclopropyl, cyclobutyl, cyclopentyl or cyclohexyl. Such groups also include, for example, cyclopropylmethyl and cyclohexylmethyl.

As used herein, the terms "alkenyl" and "alkynyl" as a group or part of a group means that the group is straight or branched. Examples of suitable alkenyl groups include vinyl and allyl. A suitable alkynyl group is acetylene or propargyl.

When used herein, the term "halogen" means fluorine, chlorine, bromine and iodine. The most preferred halogens are fluorine and chlorine, especially chlorine.

Examples of 6-membered saturated rings are morpholine, piperidine and piperazine.

Examples of 6-membered heteroaromatic rings are pyridine, pyrimidine, pyrazine, pyridazine and triazine.

Examples of 5-membered heteroaromatic rings are thiophene, furan, pyrrole, imidazole, pyrazole, oxazole, isoxazole, thiazole, isothiazole, 1,2,3-triazole, 1,2,4-triazole, oxadiazole, thiadiazole and tetrazole.

Examples of 9- or 10-membered fused bicyclic heteroaromatic rings include benzofuran, benzothiophene, benzimidazole, benzoxazole, benzothiazole, benzisothiazole, quinoline, isoquinoline and cinnoline.

In a further aspect of the present invention, the compounds of formula I may be prepared in the form of a pharmaceutically acceptable salt, especially an acid addition salt.

For use in medicine, the salts of the compounds of formula I will be non-toxic pharmaceutically acceptable salts. Other salts may, however, be useful in the preparation of the compounds according to the invention or of their non-toxic pharmaceutically acceptable salts. Suitable pharmaceutically acceptable salts of the compounds of this invention include acid addition salts which may, for example, be formed by mixing a solution of the compound according to the invention with a solution of a pharmaceutically acceptable acid such as hydrochloric acid, fumaric acid, p-toluenesulphonic acid, maleic acid, succinic acid, acetic acid, citric acid, tartaric acid, carbonic acid, phosphoric acid, sulphuric acid or benzenesulfonic acid. Preferred pharmaceutically acceptable salts of the compounds of the present invention are the hydrochloride salts. Salts of amine groups may also comprise quaternary ammonium salts in which the amino nitrogen atom carries a suitable organic group such as an alkyl, alkenyl, alkynyl or aralkyl moiety. Furthermore, where the compounds of the invention carry an acidic moiety, suitable pharmaceutically acceptable salts thereof may include metal salts such as alkali metal salts, e.g. sodium or potassium salts; and alkaline earth metal salts, e.g. calcium or magnesium salts.

The salts may be formed by conventional means, such as by reacting the free base form of the compound of formula I with one or more equivalents of the appropriate acid in a solvent or medium in which the salt is insoluble, or in a solvent such as water which is removed *in vacuo* or by freeze drying or by exchanging the anions of an existing salt for another anion on a suitable ion exchange resin.

The present invention also includes within its scope N-oxides of the compounds of formula I above. In general, such N-oxides may be formed on any available nitrogen atom. The N-oxides may be formed by conventional means, such as reacting the compound of formula I with oxone in the presence of wet alumina.

The present invention includes within its scope prodrugs of the compounds of formula I above. In general, such prodrugs will be functional derivatives of the compounds of formula I which are readily convertible *in vivo* into the required compound of formula I. Conventional procedures for the selection and preparation of suitable prodrug derivatives are described, for example, in "Design of Prodrugs", ed. H. Bundgaard, Elsevier, 1985.

A prodrug may be a pharmacologically inactive derivative of a biologically active substance (the "parent drug" or "parent molecule") that requires transformation within the body in order to release the active drug, and that has improved delivery properties over the parent drug molecule. The transformation *in vivo* may be, for example, as the result of some metabolic process, such as chemical or enzymatic hydrolysis of a carboxylic, phosphoric or sulphate ester, or reduction or oxidation of a susceptible functionality.

The present invention includes within its scope solvates of the compounds of formula I and salts thereof, for example, hydrates.

The compounds according to the invention may have one or more asymmetric centres, and may accordingly exist both as enantiomers and as diastereoisomers. It is to be understood that all such isomers and mixtures thereof are encompassed within the scope of the present invention. Furthermore, the compounds of formula I may also exist in tautomeric forms and the invention includes within its scope both mixtures and separate individual tautomers.

The compounds may exist in different isomeric forms, all of which are encompassed by the present invention.

The present invention further provides pharmaceutical compositions comprising one or more compounds of formula I in association with a pharmaceutically acceptable carrier or excipient.

Preferably the compositions according to the invention are in unit dosage forms such as tablets, pills, capsules, powders, granules, sterile parenteral solutions or suspensions, metered aerosol or liquid sprays, drops, ampoules, auto-injector devices, suppositories, creams or gels; for oral, parenteral, intrathecal, intranasal, sublingual, rectal or topical administration, or for administration by inhalation or insufflation. Oral compositions such as tablets, pills, capsules or wafers are particularly preferred. For preparing solid compositions such as tablets, the principal active ingredient is mixed with a pharmaceutical carrier, e.g. conventional tabletting ingredients such as corn starch, lactose, sucrose, sorbitol, talc, stearic acid, magnesium stearate, dicalcium phosphate or gums, and other pharmaceutical diluents, e.g. water, to form a solid pre-formulation composition containing a homogeneous mixture of a compound of the present invention, or a pharmaceutically acceptable salt thereof. When referring to these pre-formulation compositions as homogeneous, it is meant that the active ingredient is dispersed evenly throughout the composition so that the composition may be readily subdivided into equally effective unit dosage forms such as tablets, pills and capsules. This solid pre-formulation composition is then subdivided into unit dosage forms of the type described above containing from 0.1 to about 500 mg of the active ingredient of the present invention. Favoured unit dosage forms contain from 1 to 500 mg, for example 1, 5, 10, 25, 50, 100, 300 or 500 mg, of the active ingredient. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer that serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the novel compositions of the present invention may be incorporated for administration orally or by injection include aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinyl-pyrrolidone or gelatin.

In the treatment of painful conditions such as those listed below, a suitable dosage level is about 1.0 mg to 15 g per day, preferably about 5.0 mg to 1 g per day, more preferably about 5 mg to 500 mg per day, especially 10 mg to 100 mg per day. The compounds may be administered on a regimen of 1 to 4 times per day.

It will be appreciated that the amount of a compound of formula I required for use in any treatment will vary not only with the particular compounds or composition selected but also with the route of administration, the nature of the condition being treated, and the age and condition of the patient, and will ultimately be at the discretion of the attendant physician.

The invention further provides a compound of formula I as defined above, or a pharmaceutically acceptable salt thereof, for use in treatment of the human or animal body. Preferably, said treatment is for a condition which is susceptible to treatment by modulation (preferably antagonism) of VR1 receptors.

The compounds of the present invention will be of use in the prevention or treatment of diseases and conditions in which pain and/or inflammation predominates, including chronic and acute pain conditions. Such conditions include rheumatoid arthritis; osteoarthritis; post-surgical pain; musculo-skeletal pain, particularly after trauma; spinal pain; myofascial pain syndromes; headache, including migraine, acute or chronic tension headache, cluster headache, temporomandibular pain, and maxillary sinus pain; ear pain; episiotomy pain; burns, and especially primary hyperalgesia associated therewith; deep and visceral pain, such as heart pain, muscle pain, eye pain, orofacial pain, for example, odontalgia, abdominal pain, gynaecological pain, for example, dysmenorrhoea, pain associated with cystitis and labour pain, chronic pelvic pain, chronic prostatitis and endometriosis; pain associated with nerve and root damage, such as pain associated with peripheral nerve disorders, for example, nerve entrapment and brachial plexus avulsions, amputation, peripheral neuropathies, tic douloureux, atypical facial pain, nerve root damage, and arachnoiditis; itching conditions including pruritis, itch due to hemodialysis, and contact dermatitis; pain (as well as broncho-constriction and inflammation) due to exposure (e.g. via ingestion, inhalation, or eye contact) of mucous membranes to capsaicin and related irritants such as tear gas, hot peppers or pepper spray; neuropathic pain conditions such as diabetic neuropathy, chemotherapy-induced neuropathy and post-herpetic neuralgia; "non-painful" neuropathies; complex regional pain syndromes; pain associated with carcinoma, often referred to as cancer pain; central nervous system pain, such as pain due to spinal cord or brain stem damage, low back pain, sciatica and ankylosing spondylitis; gout; scar pain; irritable bowel syndrome; inflammatory bowel disease; urinary incontinence including bladder detrusor hyper-reflexia and bladder hypersensitivity; respiratory diseases including cough, chronic obstructive pulmonary disease (COPD), chronic bronchitis, cystic fibrosis, asthma and rhinitis, including allergic rhinitis such as seasonal and perennial rhinitis, and non-allergic rhinitis; autoimmune diseases; immunodeficiency disorders; and hot flushes. The compounds of the present invention may also be used to treat depression. They may also be used to treat gastro-oesaphageal reflux disease (GERD), preferably including the pain associated with GERD.

Thus, according to a further aspect, the present invention provides a compound of formula I for use in the manufacture of a medicament for the treatment or prevention of physiological disorders that may be ameliorated by modulating VR1 activity.

According to a further or alternative aspect, the present invention provides a compound of formula I for use in the manufacture of a medicament for the treatment or prevention of a disease or condition in which pain and/or inflammation predominates.

According to a further aspect of the present invention, it may be desirable to treat any of the aforementioned conditions with a combination of a compound according to the present invention and one or more other pharmacologically active agents suitable for the treatment of the specific condition. The compound of formula I and the other pharmacologically active agent(s) may be administered to a patient simultaneously, sequentially or in combination.

Thus, for example, for the treatment or prevention of pain and/or inflammation, a compound of the present invention may be used in conjunction with other analgesics, such as acetaminophen (paracetamol), aspirin and other NSAIDs, including selective cyclooxygenase-2 (COX-2) inhibitors, as well as opioid analgesics, especially morphine, NR2B antagonists, bradykinin antagonists, anti-migraine agents, anticonvulsants such as oxcarbazepine and carbamazepine, antidepressants (such as TCAs, SSRIs, SNRIs, substance P antagonists, etc.), spinal blocks, gabapentin, pregabalin and asthma treatments (such as ϑ₂-adrenergic receptor agonists or leukotriene D₄ antagonists (e.g. montelukast).

Specific anti-inflammatory agents include diclofenac, ibuprofen, indomethacin, nabumetone, ketoprofen, naproxen, piroxicam and sulindac, etodolac, meloxicam, rofecoxib, celecoxib, etoricoxib, parecoxib, valdecoxib and tilicoxib. Suitable opioid analgesics of use in conjunction with a compound of the present invention include morphine, codeine, dihydrocodeine, diacetylmorphine, hydrocodone, hydromorphone, levorphanol, oxymorphone, alfentanil, buprenorphine, butorphanol, fentanyl, sufentanyl, meperidine, methadone, nalbuphine, propoxyphene and pentazocine; or a pharmaceutically acceptable salt thereof. Suitable anti-migraine agents of use in conjunction with a compound of the present invention include CGRP-antagonists, ergotamines or 5-HT₁ agonists, especially sumatriptan, naratriptan, zolmatriptan or rizatriptan.

Thus, for example, for the treatment or prevention of cough, a compound of the present invention may be used in conjunction with other medication designed to treat this condition, such as antibiotics, anti-inflammatory agents, cystinyl leukotrienes, histamine antagonists, corticosteroids, opioids, NMDA antagonists, proton pump inhibitors, nociceptin, neurokinin (NK1, NK2 and NK3) and bradykinin (Bk1 and Bk2) receptor antagonists, cannabinoids, blockers of Na+-dependent channels and large conductance Ca(2+)-dependent K+-channel activators. Specific agents include dexbrompheniramine plus

pseudoephedrine, loratadine, oxymetazoline, ipratropium, albuterol, beclomethasone, morphine, codeine, pholcodeine and dextromethorphan.

Thus, for example, for the treatment or prevention of urinary incontinence, a compound of the present invention may be used in conjunction with other medication designed to treat this condition, such as estrogen replacement therapy, progesterone congeners, electrical stimulation, calcium channel blockers, antispasmodic agents, cholinergic antagonists, antimuscarinic drugs, tricyclic antidepressants, SNRIs, beta adrenoceptor agonists, phosphodiesterase inhibitors, potassium channel openers, nociceptin/orphanin FQ (OP4) agonists, neurokinin (NK1 and NK2) antagonists, P2X3 antagonists, musculotrophic drugs and sacral neuromodulation. Specific agents include oxybutinin, emepronium, tolterodine, flavoxate, flurbiprofen, tolterodine, dicyclomine, propiverine, propantheline, dicyclomine, imipramine, doxepin, duloxetine and 1-deamino-8-D-arginine vasopressin.

Therefore, in a further aspect of the present invention, there is provided a pharmaceutical composition comprising a compound of the present invention and an analgesic, together with at least one pharmaceutically acceptable carrier or excipient.

In a further or alternative aspect of the present invention, there is provided a product comprising a compound of the present invention and an analgesic as a combined preparation for simultaneous, separate or sequential for use in the treatment or prevention of a disease or condition in which pain and/or inflammation predominates.

Compounds of formula I can be made by reacting a compound of formula II with a compound of formula III:

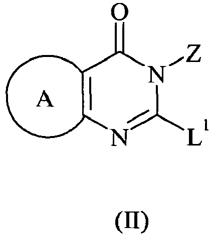

H-W (III)

wherein A, W and Z are as defined above and L¹ is a leaving group such as chlorine or bromine. The reaction is generally carried out in a solvent such as acetonitrile in the presence of a base such as potassium carbonate at about 50 to 80°C for from about 18 to 96 hours. The reaction may also be carried out in a solvent such as tetrahydrofuran in the presence of a base such as triethylamine at about 150°C. If necessary, the product is acidified using an acid such as HCl in a solvent such as ethanol to produce the salt.

Compounds of formula II can be made by reacting a compound of formula (IV): 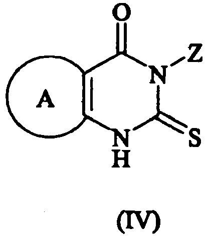 wherein A and Z are as defined above, with a chlorinating agent such as PCl₅ generally in the presence of POCl₃ at about 100°C for around 24 hours, or POCl₃ or POBr₃ at about 105 to 140°C for from about 4 to 48 hours.

Compounds of formula IV can be made by reacting a compound of formula V with a compound of formula VI:

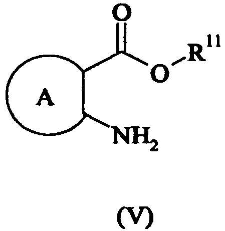

Z-NCS (VI)

wherein A and Z are as defined above and R¹¹ is a C₁₋₆alkyl group such as methyl or ethyl. The reaction is generally carried out in a solvent such as acetonitrile at reflux for from about 2 to 18 hours.

Compounds of formula IV can alternatively be prepared by reacting a compound of formula VII: 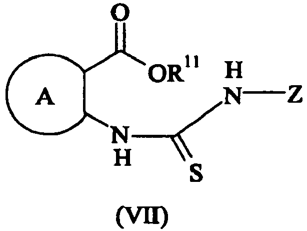 wherein A, R¹¹ and Z are as defined above, with a base such as potassium hydroxide or sodium hydroxide, generally in a solvent such as water at from about 80 to 90°C for about 30 minutes to 18 hours.

Compounds of formula VII can be prepared by reacting a compound of formula V with a compound of formula VI in the presence of a pyridine solvent at about 45°C for around 18 hours. The reaction may also be carried out in a solvent such as acetonitrile at about 50 to 80°C for around 18 to 24 hours and a catalyst such as 4-dimethylaminopyridine may also be added

Where the synthesis of intermediates and starting materials is not described these compounds are commercially available or can be made from commercially available compounds by standard methods, or by extension from the Descriptions and Examples herein.

Compounds of formula I may be converted to other compounds of formula I by known methods or by methods described in the Descriptions and Examples.

During any of the above synthetic sequences it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene and P.G.M. Wuts, Protective Groups in Organic Synthesis, John Wiley & Sons, 1991. The protecting groups may be removed at a convenient subsequent stage using methods known from the art.

The following Examples serve to illustrate the preparation of compounds of the present invention.

### Description 1 3-(4-Chlorophenyl)-2-thioxo-2,3-dihydropyrido[3,2-d]pyrimidin-4(1H)-one

A solution of 4-chlorophenyl isothiocyanate (1.10 g, 6.48 mmol) and ethyl 3-aminopyridine-2-carboxylate (J. Chem. Soc. 1956, 1045) (1.07 g, 6.48 mmol) in acetonitrile (30 ml) was heated at reflux for 2 h, then cooled to room temperature. The solid was collected by filtration, washed with cold acetonitrile (5 ml) and dried to give the title compound as a white crystalline solid (84 mg, 4.5 %). The filtrate was re-heated to reflux for 18 h and then cooled to room temperature to give a second crop of crystals. The crystals were collected by filtration, washed with acetonitrile (5 ml) and dried to give the title compound (350 mg, 19 %). ¹H NMR (400 MHz, DMSO) δ 13.09 (1 H, br. s), 8.60 (1 H, dd, *J*4.3, 1.5), 7.82 (1 H, dd, *J* 8.4, 1.5), 7.77 (1 H, dd, *J* 8.4, 4.3), 7.55 (2 H, d, *J* 8.0), 7.35 (2 H, d, *J* 8.0). *M*/*z* (ES⁺) 290, 292 (M+H⁺).

### Description 2 2-Chloro-3-(4-chlorophenyl)pyrido[3,2-d]pyrimidin-4(3H)-one

A solution of Description 1 (123 mg, 0.43 mmol) and phosphorous pentachloride (134 mg, 0.65 mmol) in phosphorous oxychloride (1 ml) was stirred at 100 °C for 24 h. The reaction mixture was cooled, evaporated *in vacuo,* and azeotroped twice with toluene. The resulting oil was then dissolved in ethyl acetate (15 ml) and washed with water (5 x 15 ml). The organic layer was dried over MgSO₄, filtered and evaporated to give a brown solid. The solid was dry loaded in acetonitrile onto silica and purified by flash column chromatography eluting with ethyl acetate/ dichloromethane (1:4) to give the title compound as a pale yellow solid (58 mg, 47 %). ¹H NMR (360 MHz, DMSO) 8 8.86 (1 H, dd, *J* 4.4, 1.6), 8.16 (1 H, dd, *J* 8.2, 1.6), 7.91 (1 H, dd, *J* 8.2, 4.4), 7.66 (2 H, d, *J* 8.7), 7.58 (2 H, d, *J* 8.7). *M*/*z* (ES⁺) 292, 294 (M+H⁺).

### Description 3 1-(4-Chlorophenyl)-9-methyl-2-thioxo-1,2,3,9-tetrahydro-6H-purin-6-one hydrochloride

To a solution of ethyl 3-nitriloalaninate *(*Synthesis, 1996, 11, 1325; 20 g, 0.156 mol) in MeCN (400 ml) was added triethylorthoformate (26 ml, 23.2 g, 0.156 mol) and the resulting solution heated to 90 °C. After 1 h the solution was cooled to room temperature and a solution of methylamine (8 M in ethanol, 20 ml, 0.156 mol) added and the reaction stirred at RT for 18 h. The reaction was condensed *in vacuo* to a viscous oil then taken up in hydrochloric acid (1 N, 180 ml). The aqueous layer was washed with dichloromethane (2 x 200 ml, 1 x 100 ml). The aqueous layer was neutralised by the addition of solid sodium bicarbonate (∼20 g) and then extracted with dichloromethane (5 x 200 ml). The organic layers were combined, dried over MgSO₄ and condensed *in vacuo* to give a brown/red solid residue. The residue was slurried in ethyl acetate (40 ml) with sonication, filtered, then the solid rinsed with ether and dried to give ethyl 5-amino-1-methyl-1*H*-imidazole-4-carboxylate (9.88 g, 37 %). Ethyl 5-amino-1-methyl-1*H*-imidazole-4-carboxylate (4.5 g, 26.6 mmol) and 4-chlorophenyl isothiocyanate (4.5 g, 26.6 mmol) were stirred in pyridine (22 ml) at 45°C for 18 h. The suspension was cooled and diluted by the addition of ice. When the ice had melted the reaction was filtered, the product rinsed with water and diethyl ether to give ethyl 5-({[(4-chlorophenyl)amino]carbonothioyl)amino)-1-methyl-1*H*-imidazole4-carboxylate. The solid was slurried in 1 % aqueous sodium hydroxide solution (15 ml) and heated at 80 °C for 30 mins. The reaction was filtered to remove insoluble impurities and then acidified to pH∼5 using hydrochloric acid (5 N), causing a thick white suspension to form. The mixture was aged for 30 minutes and filtered. The solid was rinsed with water then diethyl ether and dried to give the title compound as a white solid (5.28 g, 68 %).¹H NMR (360 MHz, DMSO) 8 7.58 (1 H, s), 7.37 (2 H, m), 7.06 (1 H, br. s), 6.96 (2 H, m), 3.54 (3 H, s). *M*/*z* (ES⁺) 293, 295 (M+H⁺).

### Description 4 1-(4-Chlorophenyl-9-ethyl-2-thioxo-1,2,3,9-tetrahydro-6H-purin-6-one hydrochloride

To a solution of ethyl 3-nitriloalaninate *(*Synthesis, 1996, 11, 1325; 25 g, 0.195 mol) in MeCN (400 ml) was added triethylorthoformate (32.5 ml, 28.9 g, 0.195 mol) and the resulting solution heated to 90 °C. After 70 min the solution was cooled to room temperature and a solution of ethylamine (2 M in tetrahydrofuran, 98 ml, 0.195 mol) added and the reaction stirred at RT for 18 h. The reaction was condensed in *vacuo* to a viscous oil then taken up in hydrochloric acid (1 N, 200 ml). The aqueous layer was washed with dichloromethane (2 x 200 ml, 1 x 100 ml). The aqueous layer was neutralised by the addition of solid sodium bicarbonate (∼25 g) and then extracted with dichloromethane (5 x 200 ml). The organic layers were combined, dried over MgSO₄ and condensed *in vacuo* to give a brown/red solid residue. The residue was slurried in ethyl acetate (50 ml), filtered, and the solid rinsed with diethyl ether and dried to give ethyl 5-amino-1-ethyl-1*H*-imidazole-4-carboxylate (13.0 g, 36 %). Ethyl 5-amino-1-ethyl-1*H*-imidazole-4-carboxylate (0.62 g, 3.4 mmol) and 4-chlorophenyl isothiocyanate (0.58 g, 3.4 mmol) were stirred in pyridine (2 ml) at 45°C for 18 h. The suspension was cooled and diluted by the addition of ice. When the ice had melted the reaction was extracted into ethyl acetate. The organic layer was dried over MgSO₄ and evaporated *in vacuo* to give a mixture of the symmetrical thiourea *N,N*-bis(4-chlorophenyl)thiourea and ethyl 5-({[(4-chlorophenyl)amino] carbonothioyl}amino)-1-ethyl-1*H-*imidazole-4-carboxylate (1.12 g). The solid was slurried in 1 % aqueous sodium hydroxide solution (20 ml) and heated at 90 °C for 16 h. The reaction was filtered, the filtrate evaporated *in vacuo,* and the residue was diluted with methanol and loaded onto a strong cation exchange (SCX) cartridge. The cartridge was washed with methanol and dichloromethane and then the product eluted with 2 M methanolic ammonia. After drying, this gave the title compound (756 mg, 72 %). ¹H NMR (400 MHz, CD₃OD) δ 7.76 (1 H, s), 7.43 (2 H, d, *J*8.5), 7.13 (2 H, d, *J*8.7), 4.16 (2 H, q, *J*7.3), 1.45 (3 H, t, *J*7.2). *Mlz* (ES⁺) 307, 309 (M+H⁺).

### Descriptions 5 2-Chloro-1-(4-chlorophenyl)-9-ethyl-1.9-dihydro-6H-purin-6-one

Description 4 (860 mg, 2.5 mmol) was suspended in a large excess of phosphorous oxychloride (>20 eq) which upon heating to 135 °C dissolved. This solution was then heated at this temperature for a further 36 h. The reaction mixture was cooled, evaporated in *vacuo,* and azeotroped twice with toluene. The resulting sticky brown oil was dissolved in dichloromethane then neutralised with sat. NaHCO₃ (aq). The dichloromethane layer was then dry loaded onto a silica flash column, eluting product with ethyl acetate/ dichloromethane (1:1) to give the title compound as a pale yellow solid (426 mg, 55 %). ¹H NMR (500 MHz, CDCl₃) δ 7.79 (1H, s), 7.52 (2 H, d, *J*8.6), 7.21 (2 H, d, *J*8.6), 4.23 (2 H, q, *J*7.3), 1.56 (3 H, t, *J*7.3*). Mlz* (ES⁺) 309, 311 (M+H⁺).

### Description 6 2-Chloro-1-(4-chlorophenyl)-9-methyl-1,9-dihydro-6H-purin-6-one

Prepared from Description 3 according to the procedure outlined in Description 5.
¹H NMR (360 MHz, DMSO) δ 8.14 (1 H, s), 7.64 (2 H, d, *J*8.6), 7.52 (2 H, d, *J*8.6), 3.76 (3 H, s). *M*/*z* (ES⁺) 295, 297 (M+H⁺).

### Description 7 3-(4-Chlorophenyl)-7-methyl-2-thioxo-2.3-dihydrothieno[3,2-d]pyrimidin-4(1H)-one

A mixture of methyl 3-amino-4-methylthiophene-2-carboxylate (5.13 g, 30 mmol) and 4-chlorophenyl isothiocyanate (5.09 g, 30 mmol) in acetonitrile (100 ml) were heated at 50 °C overnight. The cooled reaction mixture was evaporated and the residue treated with 1% NaOH solution (100 ml) and heated at 80°C for 1 h. An oil had formed in the bottom of the reaction, this was removed by cooling the mixture and dissolving the oil in dichloromethane (50 ml), and separating the organic layer. The aqueous layer was neutralized by the addition of 1 N HCl and the resulting white precipitate removed by filtration and dried in *vacuo* to give the title compound as a white solid (1.3 g, 14 %). ¹H NMR (400 MHz, DMSO) δ 13.29 (1 H, br. s), 7.85 (1 H, d, *J* 1.0), 7.53 (2 H, m), 7.30 (2 H, m), 2.34 (3 H, s). *M*/*z* (ES⁺) 309, 311 (M+H⁺).

### Description 8 2-Chloro-3-(4-chlorophenyl)-7-methylthieno[3,2-d]pyrimidin-4(3H)-one

A mixture of Description 7 (1.1 g, 3.56 mmol) and phosphorous oxychloride (16.6 ml, 178 mmol) was heated at 105 °C for 4 h. The mixture was allowed to cool, and the excess phosphorous oxychloride removed by evaporation. The residue was taken up in dichloromethane (100 ml), ice (100 g) added and the resulting mixture stirred for 30 min. The organic layer was separated, dried over Na₂SO₄, filtered and evaporated to give a dark solid (1.0 g, 90 %). ¹H NMR (400 MHz, CDCl₃) 7.51 (3 H, m), 7.22 (2 H, m), 2.41 (3 H, d, *J*1.1*). Mlz* (ES⁺) 311 (M+H⁺).

### Description 9 1-(4-Fluorophenyl)-9-methyl-2-thioxo-1,2,3,9-tetrahydro-6H-purin-6-one hydrochloride

The title compound was prepared from ethyl 3-nitriloalaninate, methylamine and 4-fluorophenyl isothiocyante, according to the procedure described in Description 3. ¹H NMR (360MHz, DMSO) δ 7.85 (1 H, s), 7.31-7.21 (4 H, m), 3.76 (3 H, s).

### Description 10 2-Chloro-1-(4-fluorophenyl)-9-methyl-1,9-dihydro-6H-purin-6-one

Prepared from Description 9 according to the procedure outlined in Description 5. ¹H NMR (CDCl₃) δ 7.75 (1 H, s), 7.23-7.21 (4 H, m), 3.83 (3 H, s). *M*/*z* (ES⁺) 279, 281 (M+H⁺).

### Description 11 6-(4-Fluorophenyl)-5-thioxo-5,6-dihydro[1,3]thiazolo[5,4-d]pyrimidin-7(4H)-one

A solution of ethyl 5-amino-1,3-thiazole-4-carboxylate *(*Tetrahedron 1985, 41, 5989; 2.0 g, 11.6 mmol), 4-fluorophenyl isothiocyanate (2.0 g, 13.1 mmol) and a catalytic quantity of 4-dimethylaminopyridine in acetonitrile (20 ml) was heated at reflux for 24 h. The reaction was partially complete. The cooled reaction mixture was filtered and the solid product collected. Without further purification this solid was added to 1 % aqueous sodium hydroxide solution (∼20 ml) and heated at 80°C for 30 min. The solution was cooled, filtered to remove scum and the filtrate acidified by adding 5 N aqueous hydrochloric acid dropwise, causing a thick white precipitate to form. The solid was collected by filtration, washed with water, then dried under vacuum to give the title compound (0.40 g, 12 %). ¹H NMR <360 MHz, DMSO) δ 13.84 (1 H, br. s), 8.91 (1 H, s), 7.32 (4 H, m). *M*/*z* (ES⁺) 280 (M+H⁺).

### Description 12 5-Chloro-6-(4-fluorophenyl)[1,3]thiazolo[5,4-d]pyrimidin-7(6H)-one

Prepared from Description 11 according to the procedure outlined in Description 5.
¹H NMR (400 MHz, CDCl₃,) δ 8.89 (1 H, s), 7.30 (4 H, m). *M*/*z* (ES⁺) 282, 284 (M+H⁺).

### Description 13 4-(9-Methyl-6-oxo-2-thioxo-2,3,6,9-tetrahydro-1H-purin-1-yl)benzonitrile hydrochloride

Prepared from ethyl 3-nitriloalaninate, methylamine and 4-cyanophenyl isothiocyanate, according to the procedure described in Description 3. ¹H NMR (500 MHz, DMSO) δ 7.96 (2 H, d, *J* 8.4), 7.88 (1 H, s), 7.46 (2 H, d, *J* 8.4), 3.77 (3 H, s). *M*/*z* (ES⁺) 284 (M+H⁺).

### Descriptions 14 4-(2-Bromo-9-methyl-6-oxo-6,9-dihydro-1H-purin-1-yl)benzonitrile

A mixture of Description 13 (0.5 g, 1.77 mmol) and phosphorous oxybromide (5 g, 17.4 mmol) was stirred at 140 °C (melt). After 48 h the reaction was cooled and the resulting waxy solid added to ice. The mixture was neutralised carefully by the addition of saturated aqueous sodium bicarbonate and solid sodium bicarbonate. The dichloromethane extracts were dried over MgSO₄ and condensed in *vacuo.* The crude product was purified by gradient flash column chromatography eluting with 5-10 % methanol in dichloromethane. A second column eluted with a 1:1 mixture of ethyl acetate and 10 % methanol in dichloromethane gave the pure title compound (55 mg, 9 %). ¹H NMR (360 MHz, CDCl₃) δ 7.86 (2 H, d, *J*8.5), 7.76 (1 H, s), 7.41 (2 H, d, *J*8.5), 3.85 (3 H, s). *Mlz* (ES⁺) 330, 332 (M+H⁺).

### Description 15 1-(3,4-Difluorophenyl)-9-methyl-2-thioxo-1,2,3,9-tetrahydro-6H-purin-6-one hydrochloride

Prepared from ethyl 3-nitriloalaninate, methylamine and 3,4-difluorophenyl isothiocyanate, according to the procedure described in Description 3. ¹H NMR (400 MHz, DMSO) δ 7.86 (1 H, s), 7.53 (1 H, q, *J* 9.4), 7.46-7.42 (1H, m), 7.12-7.06 (1 H, m), 3.76 (3 H, s). *M*/*z* (ES⁺) 295 (M+H⁺).

### Description 16 2-Bromo-1-(3.4-difluorophenyl)-9-methyl-1,9-dihydro-6H-purin-6-one

Prepared from Description 15 according to the procedure described in Description 14. ¹H NMR (400 MHz, DMSO) δ 8.12 (1 H, s), 7.78-7.74 (1 H, m), 7.66 (1 H, q, *J* 9.4), 7.43-7.37 (1 H, m), 3.76 (3 H, s). *M*/*z* (ES⁺) 341, 343 (M+H⁺).

### Description 17 3-(Trifluoromethyl)-5,6,7,8-tetirahydro-1,6-naphthyridine

The title compound was prepared according to the procedure described in WO-A-03093266.

### Description 18 2-(Trifluoromethyl)-5,6,7,8-tetrahydro-1,7-naphthyridine

The title compound was prepared according to the procedure described in WO-A-04069162.

### Description 19 2-(Trifluoromethyl)-5,6,7,8-tetrahydropyrido[3,4-d]pyrimidine

The title compound was prepared according to the procedure described in WO-A-44007468.

### Description 20 2-(Trifluoromethyl)4,5,6,7-tetrahydro[1.3]thiazolo[4,5-c]pyridine hydrochloride

The title compound was prepared according to the procedure described in WO-A-04064778.

### Description 21 2-(Trifluoromethyvl)-5,6,7,8-tetrahvdroimidazo[1,2-a]pyrazine hydrochloride

The title compound was prepared according to the procedure described in WO-A-03004498.

### Description 22 3-(Trifluoromethyl)pyrrolidine

The title compound was prepared according to the procedure described in WO-A-04005295.

### Descriptions 23 3-(3-Ethyl-1,2,4-oxadiazol-5-piperidine

The title compound was prepared according to the procedure described in EP 459568.

### Example 1 1-(4-Chlorophenyl)-9-methyl-2-piperidin-1-yl-1,9-dihydro-6H-purin-6-one

A mixture of Description 6 (76 mg, 0.26 mmol), piperidine (39 µl, 0.39 mmol) and potassium carbonate (177 mg, 1.28 mmol) in acetonitrile (anhydrous, 3 ml) was heated at 60 °C for 18 h, then cooled to room temperature. The reaction mixture was evaporated in *vacuo,* dissolved in water and dichloromethane added and the mixture vortexed. After settling, the mixture was added to a phase separation cartridge and the dichloromethane phase was separated and concentrated. The resulting solid was washed with diethyl ether to give the title compound as a white solid (57 mg, 64 %). ¹H NMR (400 MHz, DMSO) δ 7.89 (1 H, s), 7.56 (2 H, d, *J* 8.7)*,* 7.42 (2 H, d, *J* 8.7)*,* 3.67 (3 H, s), 3.00-2.98 (4 H, m), 1.38-1.35 (2 H, m), 1.19-1.16 (4 H, m). *M*/*z* (ES⁺) 344, 346 (M+H⁺).

### Examples 2 1-(4-Chlorophenyl)-9-methyl-2-(2-methylpyrrolidin-1-yl)-1,9-dihydro-6H-purin-6-one hydrochloride

A mixture of Description 6 (100 mg, 0.34 mmol), 2-methylpyrrolidine (35 µl, 0.34 mmol) and potassium carbonate (242 mg, 1.75 mmol) in acetonitrile (anhydrous, 3 ml) was heated at 70°C for 24 h, then cooled to room temperature. The reaction mixture was evaporated in *vacuo,* dissolved in water and dichloromethane added and the mixture vortexed. After settling, the mixture was added to a phase separation cartridge and the dichloromethane phase was separated and concentrated. The resulting solid was washed with diethyl ether to give pure compound as the free base (89 mg, 025 mmol). The hydrochloride salt was made by dissolving the solid in ethanol (2 ml), adding hydrochloric acid (aq. 2N HCl, 250 µl, 0.5 mmol) and warming the solution with a heat gun. This was evaporated in *vacuo,* azeotroped with ethanol, triturated with diethyl ether, and the solid collected by filtration and dried to give the title compound (89 mg, 67 %). ¹H NMR (400 MHz, CD₃OD) δ 8.98 (1 H, s), 7.58 (2 H, s), 7.54 (1H, d, *J*8.5), 7.17 (1H, d, *J*8.4), 4.30-4.22 (1 H, m), 3.90 (3 H, s), 3.18-3.13 (1 H, m), 2.40-2.34 (1 H, m), 2.12-2.08 (1 H, m), 1.80-1.74 (1 H, m), 1.70-1.58 (1 H, m), 1.50-138 (1 H, m), 1.34 (3 H, d, *J* 6.0). *Mlz* (ES⁺) 344, 346 (M+H⁺).

Examples 3-38 were prepared using the appropriate chloro or bromo pyrimidinone core (Descriptions 2, 5, 6, 8, 10, 12, 14, 16) and the appropriate amine in a procedure analogous to Example 1. The substituted amines used are commercially available or described in Descriptions 17-23. Where the product did not precipitate analytically pure from the reaction it was purified by recrystallisation, flash column chromatography, preparative thin layer chromatography or mass directed HPLC as appropriate.

| **EX** | **NAME** | **M/z ES⁺ [M+H⁺]** | **¹H NMR** |
|---|---|---|---|
| 3 | 1-(4-Chlorophenyl)-9-methyl-2-pyrrolidin-1-yl-1,9-dihydro-6*H-*purin-6-one | 330, 332 | (400 MHz, CD₃OD) δ 7.76 (1 H, s), 7.54 (2 H, d, *J* 8.7), 7.37 (2 H, d, *J* 8.7), 3.78 (3 H, s), 3.14-3.11 (4 H, m), 1.80-1.76 (4 H, m). |
| 4 | 1-(4-Chlorophenyl)-9-methyl-2-morpholin-4-yl-1,9-dihydro-6*H*-purin-6-one | 346,348 | (400 MHz, DMSO) δ 7.92 (1 H, s), 7.57 (2 H, d, *J* 8.7), 7.45 (2 H, d, *J* 8.7), 3.69 (3 H, s), 333-3.31 (4 H, m), 3.00-2.98 (4 H, m). |
| 5 | 1-(4-Chlorophenyl)-9-methyl-2-[4-(trifluoromethyl)piperidin-1-yl]-1,9-dihydro-6*H*-purin-6-one | 412,414 | (400 MHz, CD₃OD) δ 7.86 (1 H, s), 7.54 (2 H, d, *J* 8.7), 7.40 (2 H, d, *J* 8.7), 3.77 (3 H, s), 3.65-3.62 (2 H, m), 2.78-2.72 (2 H, m), 2.30-2.20 (1 H, m), 1.69-1.67 (2 H, m), 1.23-1.13 (2 H,m). |
| 6 | 1-(4-Chlorophenyl)-9-methyl-2-[3-(trifluoromethyl)piperidin-1-yl]-1,9-dihydro-6*H*-purin-6-one | 412, 414 | (500 MHz, CD₃OD) δ 7.90 (1H,s),7.57(2 H,d,*J* 8.9), 7.43 (2 H, br. s), 3.80-3.79 (4 H, m), 3.53-3.49 (1 H, m), 2.79 (1 H, *J* 11*.*8)*,* 2.70-2.64 (1H, m), 2.17-2.09 (1 H, m), 1.92-1.90 (1 H, m), 1.61-1.58 (1 H, m), 1.44-1.36 (1 H, m), 1.21-1.15 (1 H, m). |
| 7 | 1-(4-Chlorophenyl)-9-ethyl-2-(3-methylpiperidin-1-yl)-1,9-dihydro-6*H*-purin-6-one | 372, 374 | (400 MHz, DMSO) δ 7.96 (1 H, s), 7.56 (2 H, d, *J* 8.8), 7.42 (2 H, d, *J* 8.7), 4.11 (2 H, q, *J* 7.3), 3.33-3.28 (2 H, m), 2.66-2.56 (1 H, m), 235-2.29 (1 H, m), 1.61-1.55 (1 H, m), 1.42 (3 H, t, *J*4.2), 1.42-136 (1 H, m), 1.18-1.16 (1 H, m), 1.01-0.85 (2 H, m), 0.67 (3 H, d, *J* 6.7). |
| 8 | 1-{4-Chlorophenyl)-2-(3,3-dimethylpiperidin-1-yl)-9-ethyl-1,9-dihydro-6*H-*purin-6-one | 386, 388 | (500 MHz, DMSO) δ 7.98 (1 H, s), 7.57 (2 H, d, *J* 8.5), 7.41 (2 H, d, *J*8.6), 4.11 (2 H, q, *J* 7.2), 2.89-2.87 (2 H, m), 2.72 (2 H, s), 1.42 (3 H, t, *J* 7.2), 1.16-1.08 (4 H, m), 0.68 (6 H, s). |
| 9 | 1-(4-Chlorophenyl)2-(4,4-difluoropiperidin-1-yl)9-ethyl-1,9-dihydro-6*H*-purin-6-one | 394,396 | (400 MHz, DMSO) δ 8.02 (1 H, s), 7.59 (2 H, d, *J* 8.7), 7.50 (2 H, d, *J* 8.7), 4.14 (2 H, q, *J* 7.2), 3.15-3.13 (4 H, m), 1.78-1.68 (4 H, m), 1.44 (3 H, t, *J* 7.3). |
| 10 | 1-(4-Chlorophenyl)-9-ethyl-2-(3-methoxypiperidin-1-yl)-1,9-dihydro-6*H*-purin-6-one | 388,390 | (400 MHz, DMSO) δ 7.96 (1 H, s), 7.57 (2 H, d, *J* 8.8), 7.53-7.38 (2 H, br. s), 4.11 (2 H, q, *J* 7.3), 3.49-3.43 (1 H, m), 328-321 (1 H, m), 3.07 (3 H, s), 2.76-2.68 (2 H, m), 2.50-2.45 (1 H, m), 1.83-1.75 (1 H, m), 1.47-1.41 (4 H, m), 1.19-1.01 (2 H, m). |
| 11 | 2-(3-Benzylpiperidin-1-yl)-1-(4-chlorophenyl)-9-ethyl-1,9-dihydro-6*H*-purin-6-one | 448,450 | (500 MHz, DMSO) δ 7.95 (1 H, s), 7.54-7.44 (1 H, m), 7.34-7.20 (6 H, m), 7.01 (2 H, d, *J* 7.0), 4.07 (2 H, q, *J* 72), 3.40-3.36 (1 H, m), 3.31-3.25 (1 H, m), 2.66 (1H, t, *J* 10.9), 2.37-2.30 (2 H, m), 223-2.18 (1 H, m), 1.56-1.53 (1 H, m), 1.46-1.38 (4 H, m), 1.20-1.13 (1 H, m), 1.13-1.06 (1 H, m), 1.00-0.93 (1 H, m). |
| 12 | 1-(4-Chlorophenyl)-9-ethyl-1,9-dihydro-2-(3-phenyl-1-piperidinyl)-6*H*-purin-6-one | 434,436 | (500 MHz, DMSO) δ 7.97 (1 H, s), 7.60 (2 H, br. s), 7.48 (2 H, d, *J* 8.8), 7.25 (2 H, t, *J* 7.4), 7.19 (1 H, t, *J* 7.4), 7.04 (2 H, d, *J* 7.5), 4.10 (2 H, q, *J* 7.2), 3.53-3.50 (1 H, m), 3.39-3.34 (2 H, m), 2.74 (1 H, t, *J* 12.1), 2.64 (1H, t, *J* 11.9), 2.25-2.16 (1 H, m), 1.75-1.69(1 H, m), 1.60-1.50 (2 H, m), 1.41 (3 H, t*, J* 7.2)*.* |
| 13 | Reference Example 3-(4-Chlorophenyl)-2-[3-(trifluoromethyl)piperidin-1-yl]pyrido[3,2-*d*]pyrimidin-4(3*H*)-one | 409, 411 | (400 MHz, DMSO) δ 8.63 (1H, dd, *J* 1.5, 4.3), 7.93 (1 H, dd, *J* 1.5, 8.2), 7.74 (1 H, dd, *J* 4.3, 8.2), 7.62-7.56 (4 H, m), 3.75-3.69 (1 H, m), 3.35-3.45 (1 H, m), 2.75-2.57 (2 H, m), 2.18-2.08 (1 H, m), 1.84-1.77 (1 H, m), 1.49-1.44 (1 H, m), 1.36-1.26 (1 H, m), 1.05-0.97 (1 H, m). |
| 14 | 1-(4-Chlorophenyl)-2-(3,3-difluoropiperidin-1-yl)-9-ethyl-1,9-dihydro-6*H*-purin-6-one | 394,396 | (400 MHz, DMSO) δ 8.01 (1 H, s), 7.58-7.56 (2 H, m), 7.44-7.40 (2 H, m), 4.13 (2 H, q, *J* 7.2), 3.41-3.35 (3 H, m), 2.98-2.95 (2 H, m), 1.92-1.82 (2 H, m), 1.43 (3 H, t, *J* 7.3 Hz), 1.22-1.12 (2 H, m). |
| 15 | 1-(4-Chlorophenyl)-9-ethyl-2-[3-(trifluoromethyl)pyrrolidin-1-yl]-1,9-dihydro-6*H*-purin-6-one | 412,414 | (400 MHz, DMSO) δ 7.89 (1 H, s), 7.5 (2 H, d, *J* 8.2), 7.46-7.43 (2 H, m), 4.08 (2 H, q, *J* 7.2), 3.45-3.38 (1 H, m), 3.15-2.93 (4 H, m), 2.01-1.97 (1 H, m), 1.84-1.74 (1 H, m), 1.41 (3 H, t, *J* 7.3 Hz). |
| 16 | 1-(4-Chlorophenyl)-9-ethyl-2-[3-(trifluoromethyl)piperidin-1-yl]-1,9-dihydro-6*H*-purin-6-one | 426, 428 | (400 MHz, DMSO) δ 7.99 (1 H,s), 7.57 (2 H, d, *J* 7.6), 7.46 (2 H, br. s), 4.15-4.07 (2 H, m), 3.67-3.61 (1 H, m), 2.71-2.62 (1 H, m), 2.62-2.54 (1 H, m), 2.11-2.05 (1 H, m), 1.82-1.76 (1 H, m), 1.49-1.41 (4 H, m), 1.34-1.24 (1 H, m), 1.09-0.97 (1 H, m). |
| 17 | Reference Example 3-(4-Chlorophenyl)-7-methyl-2-[3-(trifluoromethyl)piperidin-1-yl]thieno[3,2-*d*]pyrimidin-4(3*H*)-one | 429,431 | (400 MHz, CDCl₃) δ 7.46 (2 H, dd, *J* 8.2, 1.1), 7.39 (1 H, d, *J* 1.1), 7.28 (2H, d, *J* 8.2), 3.78 (1 H, m), 3.39 (1 H, br. d, *J* 12.4), 2. 78 (1 H, dd, *J* 12.7, 11.0), 2.61 (1 H, m), 2.35 (3 H, d, *J* 1.0), 2.12 (1 H,m), 1.91 (1 H, m), 1.56 (1 H, m), 1.35 (1 H, m), 1.17 (1H, m). |
| 18 | Reference Example 3-(4-Chlorophenyl)-7-methyl-2-[3-(trifluoromethyl)-7,8-dihydro-1,6-naphthyridin-6(5*H*)-yl]thieno[3,2-*d*]pyrimidin-4(3*H*-one | 479,481 | (500 MHz, CDCl₃) δ 8.66 (1 H, s), 7.65 (1 H, s), 7.46 (2 H, d, *J* 8.1), 7.42 (1 H, s), 7.33 (2 H, d, *J* 8.1), 4.49 (2 H, s), 3.40 (2 H, t, *J* 5.0), 2.61 (2 H, m), 2.37 (3 H, s). |
| 19 | 2-Azetidin-1-yl-1-(4-chlorophenyl)-9-ethyl-1,9-dihydro-6*H*-purin-6-one | 330,332 | (500 MHz, CDCl₃) δ 7.54 (1 H, s), 7.46 (2 H, d, *J* 8.6), 7.25 (2 H, d, *J* 8.3), 4.09 (2 H, q, *J* 7.3), 3.61 (4 H, t, *J* 7.6), 2.09-2.03 (2 H, m), 1.50 (3 H, t, *J* 7.3). |
| 20 | 1-(4-Chlorophenyl)-9-methyl-2-[2-(trifluoromethyl)-5,8-dihydropyrido[3,4-*d*]pyrimidin-7(6*H*)-yl]-1,9-dihydro-6*H*-purin-6-one | 462,464 | (400 MHz, CDCl₃) δ 8.58 (1 H, s), 7.66 (1 H, s), 7.49 (2 H, d, *J* 8.6), 7.32 (2 H, d, *J* 8.6), 4.5 9 (2 H, s), 3.76 (3 H, s), 3.34 (2 H, t, *J* 5.6), 2.40 (2 H, t, *J* 5.3). |
| 21 | 1-(4-Chlorophenyl)-9-ethyl-2-[2-(trifluoromethyl)-6,7-dihydro[1,3]thiazolo[4,5-c]pyridin-5(4*H*)-yl]-1,9-dihydro-6*H*-purin-6-one | 481,483 | (400 MHz, CDCl₃) δ 7.65 (1 H, s), 7.49-7.47 (2 H, m), 734-732 (2 H, m), 4.45 (2 H, s), 4.15 (2 H, q, *J* 7.3), 3.42 (2 H, t, *J* 5.5), 2.48 (2 H, s), 1.53 (3 H, t, *J* 7.3). |
| 22 | 4-{9-Methyl-6-oxo-2-[3-(trifluoromethyl)piperidin-1-yl]-6,9-dihydro)-1*H*-purin-1-yl}benzonitrile | 403 | (500 MHz, CDCl₃) δ 7.80 (2 H, d, *J* 8.7), 7.63 (1 H, s), 7.48 (2H, d, *J* 8.1), 3.76 (3 H, s), 3.71 (1 H, m), 3.30 (1 H, m), 2.80 (1 H, m), 2.63-2.57 (1 H, m), 2.07 (1 H, m), 1.92 (1 H, m), 1.58 (1 H, m), 1.38 (1 H, m), 1.08 (1 H, m). |
| 23 | 1-(4-Chlorophenyl)-9-methyl-2-[2-(trifluoromethyl)-5,8-dihydro-1,7-naphthyridin-7(6*H*)-yl]-1,9-dihydro-6*H*-purinr6-one | 461,463 | (400 MHz, DMSO) δ 7.93 (1 H, s), 7.80 (1 H, d, *J* 7.9), 7.67 (1 H, d, *J* 8.0), 7.56 (2 H**,** d, *J* 8.8), 7.51 (2 H**,** d, *J* 6.7), 4.44 (2 H**,** s), 3.72 (3 H, s), 3.34-328 (2 H, m), 2.32-2.28 (2 H, m). |
| 24 | 1-(3,4-Difluorophenyl)-9-methyl-2-[3-(trifluoromethyl)piperidin-1-yl]-1,9-dihydro-6*H*-purin-6-one | 414 | (500 MHz, DMSO) δ 7.94 (1 H, s), 7.78-7.65 (1 H, m), 7.60-7.54 (1 H, m), 7.50-7.28 (1 H, m), 3.69 (3 H, s), 3.67-3.64 (1 H, m), 3.38-330 (1 H, m), 2.78-2.68 (1 H, m), 2.61 (1 H, t, *J* 11.9), 2.23-2.07 (1 H, m), 1.83-1.77 (1 H, m), 1.55-1.45 (1 H, m), 1.34-1.27 (1 H, m), 1.09-0.97 (1 H, m). |
| 25 | 1-(4-Chlorophenyl)-9-methyl-2-[3-(trifluoromethyl)-7,8-dihydro-1,6-naphthyridin-6(5*H*)-yl]-1,9-dihydro-6*H*-purin-6-one | 461,463 | (500 MHz, CDCl₃) δ 8.67 (1 H, s), 7.63 (2 H, s), 7.46 (2 H, d, *J* 8.6), 7.31 (2 H, d, *J* 8.5), 4.46 (2H, s), 3.76 (3 H, s), 3.41 (2 H, t, *J* 5.8), 2.60 (2 H, t, *J* 5.7). |
| 26 | Reference Example 6-(4-Fluorophenyl)-5-[3-(trifluoromethyl)-7,8-dihydro-1,6-naphthyridin-6(5*H*)-yl][1,3]thiazolo[5,4-*d*]pyrimidin-7(6*H*)-one | 448 | (500 MHz, CDCl₃) δ 8.66 (2 H, m), 7.61 (1 H, s), 7.40-7.38 (2 H, m), 7.21 (2 H, m), 4.50 (2 H, s), 3.48 (2 H, t, *J* 5.8), 2.61 (2 H, t, *J* 5.7). |
| 27 | 1-(4-Fluorophenyl)-9-methyl-2-[3-(trifluoromethyl)piperidin-1-yl]-1,9-dihydro-6*H*-purin-6-one | 396 | (360 MHz,CDCl₃) δ 7.63 (1 H, s), 7.29 (2 H, m), 7.18 (2 H, t, *J* 8.6), 3.75 (3 H, s), 3.40 (1 H, d, *J* 13.4), 2.75 (1 H, t, *J* 11.8), 2.64-2.56 (1 H, m), 2.09-2.01 (1 H, m), 1.91 (1 H, d, *J* 13.5), 1.57 (2 H, d, *J* 13.4), 1.42-1.32 (1 H, m), 1.17-1.09 (1 H, m). |
| 28 | 1-(4-Chlorophenyl)-9-ethyl-2-[3-(trifluoromethyl)-7,8-dihydro-1,6-naphthyridin-6(5*H*)-yl]-1,9-dihydro-6H-purin-6-one | 475 | (400 MHz, CDCl₃) δ 8.66 (1 H, s), 7.65 (1H, s), 7.63 (1 H, s), 7.45 (2 H, d, *J* 8.5), 7.31 (2 H, d, *J* 8.5), 4.44 (2 H, s), 4.16 (2 H, q, *J* 7.3), 3.41 (2 H, t, *J* 5.7)*,* 2.61 (2 H, t, *J* 5.2), 1.53 (3 H, t, *J* 7.3). |
| 29 | 1-(4-Chlorophenyl)-9-ethyl-2-[3-(4methyl-4*H*-1,2,4-triazol-3-yl)piperidin-1-yl]-1,9-dihydro-6*H*-purin-6-one | 439,441 | (500 MHz, DMSO) δ 8.35 (1 H, s), 7.98 (1 H, s), 7.58-7.54 (2 H, m), 7.50-7.46 (2 H, m), 4.12 (2 H, q, *J* 7.2), 3.64-3.60 (1 H, m), 3.55 (3 H, s), 2.91 (1 H, t, *J* 11.8), 2.73-2.63 (2 H, m), 1.89-1.84 (1 H, m), 1.56-1.50 (2 H, m), 1.42 (3 H, t, *J* 7.2), 1.20-1.12 (2 H, m). |
| 30 | 1-(4-Chlorophenyl)-9-ethyl-2-[3-(3-ethyl-1,2,4-oxadiazol-5-yl)piperidin-1-yl]-1,9-dihydro-6*H*-purin-6-one | 454,456 | (500 MHz, DMSO) δ 7.99 (1 H, s), 7.60-7.17 (4 H, m), 4.12 (2 H, q, *J* 7.2), 3.63-3.59 (1 H, m), 3.32-3.28 (1 H, m), 3.12-3.06 (2 H, m), 2.86-2.82 (1 H, m), 2.69 (2 H, q, *J* 7.5), 1.94-1.89 (1 H, m), 1.78-1.72 (1 H, m),1.42 (3 H, t, *J* 7.2), 1.28-1.22 (1 H, m), 1.20 (3 H, t, *J* 6.9), 1.15-1.08 (1 H, m). |
| 31 | 1-(4-Chlorophenyl)-2-(3,4-dihydroisoquinolin-2(1*H*-yl)9-ethyl-1,9-dihydro-6*H*-purin-6-one | 406,408 | (500 MHz, DMSO) δ 7.99 (1 H, s), 7.55 (2 H, d, *J* 8.6), 7.48 (2 H, d, *J* 8.6), 7.14-7.10 (3 H, m), 7.04-7.00 (1 H, m), 4.33 (2 H, s), 4.15 (2 H, q, *J* 7.2), 3.22 (2 H, t, *J 5*.6), 2.24 (2 H, t, *J* 5.3),1.45 (3 H, t, *J* 7.3). |
| 32 | 1-(4-Chlorophenyl)-2-(6,7-dihydrothieno[3,2-*c*]-5(4*H*)-yl)-9-ethyl-1,9-dihydro-6*H*-purin-6-one | 412, 414 | (400 MHz, DMSO) δ 7.98 (1 H, s), 7.57 (2 H, d, *J* 8.6), 7.48 (2 H, d, *J* 8.6), 7.28 (1 H, d, *J* 5.0), 6.84 (1 H, d, *J* 5.1), 4.22 (2 H, s), 4.14 (2 H, q, *J* 7.2), 3.34-3.28 (2 H, m), 2.25-2.18 (2 H, m), 1.44 (3 H, t, *J* 7.2). |
| 33 | 1-(4-Chlorophenyl)-9-methyl-2-(3-phenylpyrrolidin-1-yl)-1,9-dihydro-6*H*-purin-6-one | 406,408 | (400 MHz, DMSO) δ 7.80 (1 H, s), 7.57-7.50 (3 H, m), 735-7.16 (6H, m), 3.62 (3 H, s), 3.60-3.57 (1 H, m), 3.26-3.22 (1 H, m), 3.14-3.10 (1 H, m), 3.06-3.00 (1 H, m), 2.95-2.92 (1 H, m), 2.09-2.08 (1 H, m), 1.84-1.74 (1 H, m). |
| 34 | 1-(4-Chlorophenyl)-9-ethyl-2-(3-phenylpyrrolidin-1-yl)-1,9-dihydro-6*H*-purin-6-one | 420, 422 | (360 MHz, DMSO) δ 7.86 (1 H, s), 7.58-7.50 (3 H, m), 7.35-7.15 (6 H, m), 4.06 (2H, q, *J* 7.2), 3.59 (1 H, dd, *J* 7.1, 10.0), 3.30-3.20 (1 H, m), 3.17-3.09 (1 H, m), 3.01 (1 H, t, *J*9.6), 2.97-2.89 (1 H, m), 2.14-2.06 (1 H, m), 1.84-1.72 (1 H, m), 1.40 (3 H, t, *J*7.3). |
| 35 | 1-(4-Chlorophenyl)-2-(7-fluoro-3,4-dihydroisoquinolin-2(1*H*)-9-methyl-1,9-dihydro-6*H*-purin-6-one | 410,412 | (400 MHz, CDCl₃) δ 7.61 (1 H, s), 7.46-7.44 (2 H, m), 7.32-7.28 (2 H, m), 6.99 (1 H, dd, *J* 5.7, 8.4), 6.87-6.83 (1 H, m), 6.78 (1 H, dd, *J* 2.5, 9.3), 4.37 (2 H, s), 3.77 (3 H, s), 3.27 (2 H, t, *J* 5.8), 2.31 (2 H, t, *J* 5.6). |
| 36 | 2-(7-Chloro-3,4-dihydroisoquinolin-2(1*H*)-yl)-1-(4-chlorophenyl)-9-methyl-1,9-dihydro-6*H*-purin-6-one | 426,428 | (400 MHz, CDCl₃) δ 7.61 (1 H, s), 7.46-7.44 (2 H, m), 7.31-7.29 (2 H, m), 7.12 (1 H, dd, *J* 2.0, 8.2), 7.07 (1 H, s), 6.97 (1 H, d, *J* 8.2), 4.35 (2 H, s), 3.76 (3 H, s), 3.27 (2 H, t, *J* 5.8), 2.32 (2 H, t, *J* 5.7). |
| 37 | 2-(7-Fluoro-3,4-dihydroisoquinolin-2(1*H*)-yl)-1-(4-fluorophenyl)-9-methyl-1,9-dihydro-6*H*-purin-6-one | 394 | (400 MHz, CDCl₃) δ 7.61 (1 H, s), 736-7.32 (2 H, m), 7.19-7.15 (2 H, m), 6.98 (1 H, dd, *J* 5.7, 8.4), 6.87-6.83 (1 H, m), 6.78 (1 H, dd, *J* 2.4, 9.2), 4.37 (2 H, s), 3.77 (3 H, s), 3.27 (2 H, t, *J* 5.8), 2.29 (2 H, t, *J* 5.6). |
| 38 | 2-(7-Chloro-3,4-dihydroisoquinolin-2(1*H*)-yl)-1-(4-fluorophenyl)-9-methyl-1,9-dihydro-6*H*-purin-6-one | 410,412 | (400 MHz, CDCl₃) δ 7.62 (1 H, s), 7.35-7.31 (2 H, m), 7.16 (2 H, dd, *J* 8.4, 8.4), 7.11 (1 H, dd, *J* 2.1, 8.2), 7.08 (1 H, s), 6.95 (1 H, d, *J* 8.2), 4.36 (2 H, s), 3.77 (3 H, s), 3.27 (2 H, t, *J* 5.8), 2.30 (2 H, t, *J* 5.7). |

### Examples 39 1-(4-Chlorophenyl)-9-ethyl-2-[2-(trifluoromethyl)-5,6-dihydroimidazo[1,2-a]pyrazin-7(8H)-yl]-1,9-dihydro-6H-purin-6-one

A solution of Description 5 (23 mg, 0.07 mmol), triethylamine (47 µl, 0.35 mmol), and Description 21 (21mg, 0.1 mmol) in anhydrous tetrahydrofuran (3 ml) was irradiated at 150 °C using a Smith Synthesizer microwave for a total of 3 hours. The reaction was purified (without work up) by preparative thin layer chromatography using 5 % methanol in dichloromethane with 0.5 % ammonia as the mobile phase to give the title compound as a white solid (21 mg, 47 %). ¹H NMR (400 MHz, DMSO) δ 8.02 (1 H, s), 7.69 (1 H, s), 7.59 (2 H, d, *J* 8.8), 7.54 (2 H, d, *J* 8.7), 4.35 (2 H, s), 4.13 (2 H, q, *J* 7.2), 3.61-3.56 (2 H, m), 3.46-3.38 (2 H, m), 1.41 (3 H, t, *J* 7.3). *M*/*z* (ES⁺) 464, 466 (M+H⁺).

The above exemplified compounds of the present invention have been tested in the following assay and generally possess an IC₅₀ < 300nM and, in the majority of cases, < 200 nM. Other assays, such as electrophysiology using rat VR1 expressed in HEK cells measuring activity at various pH levels, can be used.

### Determination of in vitro activity

*In vitro* activity of compounds was measured using one or both of the following assays.

### Method I

CHO cells, stably expressing recombinant rat or human VR1 receptors and plated into black-sided 384-well plates, were washed three times with assay buffer (containing Hepes, NaCl₂, KCl, MgCl₂, CaCl₂, sucrose, glucose and probenecid, pH 7.4) and then incubated with test compound and 4uM Fluo-3-AM for 60 minutes at room temperature in darkness. Cells were washed three times more to remove excess dye, before being placed, along with plates containing capsaicin and test compounds into a Molecular Devices FLIPR³⁸⁴. The FLIPR³⁸⁴ simultaneously performed automated pharmacological additions and recorded fluorescence emission from Fluo-3. In all experiments, basal fluorescence was recorded, before re-addition of test compounds and subsequent addition of a previously determined concentration of capsaicin that evoked 80% of the maximum response. Inhibition of capsaicin evoked increases in intracellular [Ca²⁺] were expressed relative to wells on the same plate to which an EC80 concentration of capsaicin was added in the absence of test compounds.

### Method 2

Antagonists were ranked by absolute efficacy at a single low concentration vs. activation by either pH 5.5 or capsaicin (500 nM) using a medium-throughput electrophysiology assay. TRPV1 activity is initially determined using a 5 second application of 500 nM capsaicin. Agonist (either pH 5.5 or capsaicin) is then applied for 5 seconds followed by a 30 second wash period until a stable control response is achieved. Inhibition of the agonist response is determined following applications of a single concentration of test compound and inhibition is monitored using repeated agonist activation in the presence of the compound until a stable inhibition state is achieved (up to a maximum of 10 minutes of application). If a successful recovery was achieved by re-applying a control wash, additional compounds can be tested sequentially. Inhibition effect of the drug is calculated as the sustained maximum current within the 5 second agonist application divided by the control sustained maximum current before the drug had been applied, multiplied by 100 (= % inhibition @ the test concentration).

## Claims

1. A compound of formula I: 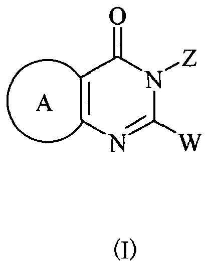 wherein:
Wis 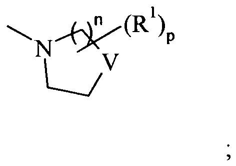
A is a five-membered heteroaromatic ring containing 2 N heteroatoms;
A is optionally substituted by one, two or three groups independently chosen from halogen, hydroxy, S(O)ᵣC₁₋₆alkyl, S(O)ᵣNR²R³, formyl, C₁₋₆alkylcarbonyl, C₁₋₆alkyl, haloC₁₋₆alkyl, hydroxyC₁₋₆alkyl, C₁₋₆alkoxy, haloC₁₋₆alkoxy, hydroxyC₁₋₆alkoxy, C₃₋₇cycloalkyl, C₃₋₇cycloalkoxy, C₂₋₆alkenyl, C₂₋₆alkynyl, amino, nitro, cyano, C₁₋₆alkylamino, di(C₁₋₆alkyl)amino, aminoC₁₋₆alkyl, aminoC₁₋₄alkoxy, C₁₋₆alkylaminoC₁₋₆alkyl, di(C₁₋₆alkyl)aminoC₁₋₆alkyl; and a ring selected from phenyl, naphthyl, a five-membered heteroaromatic ring containing one, two, three or four heteroatoms independently chosen from O, N or S, at most one heteroatom being O or S, and a six-membered heteroaromatic ring containing one, two or three N atoms, the ring being optionally substituted by halogen, hydroxy, cyano, nitro, NR²R³ as defined below, C₁₋₆alkyl, C₂₋₆alkenyl, C₂₋₆alkynyl, haloC₁₋₆alkyl, C₁₋₆alkoxy, haloC₁₋₆alkoxy, C₃₋₇cycloalkyl or hydroxyC₁₋₆alkyl;
R¹ is X-Y-P⁴;
each R² and R³ is independently hydrogen or C₁₋₆alkyl or R² and R³, together with the nitrogen atom to which they are attached, may form a saturated 4-7 membered ring;
n is zero, one, two or three;
when n is zero or one, V is CH₂;
when n is two or three, V is CH₂, O or NR⁵;
when V is CH₂, the bond formed by V and an adjacent carbon ring atom is optionally fused to a phenyl ring, a five-membered heteroaromatic ring containing 1, 2 or 3 heteroatoms independently chosen
when V is CH₂, the bond formed by V and an adjacent carbon ring atom is optionally fused to a phenyl ring, a five-membered heteroaromatic ring containing 1, 2 or 3 heteroatoms independently chosen from O, N and S, providing that no more than one O or S is present, or a six-membered heteroaromatic ring containing 1, 2 or 3 N atoms; the ring being optionally substituted by one or more R¹ groups;
R⁵ is hydrogen or together with an adjacent N-C ring bond forms a fused five-membered heteroaromatic ring containing one, two, three or four nitrogen atoms optionally substituted by one or more R¹ groups;
X is a bond, O or NR⁶;
Y is (CR⁷R⁸)ₐ;
each R⁴ is independently halogen, hydroxy, cyano, C₁₋₆alkyl, haloC₁₋₆alkyl, hydroxyC₁₋₆alkyl, C₁₋₆alkoxy, haloC₁₋₆alkoxy, hydroxyC₁₋₆alkoxy, C₃₋₇cycloalkyl, C₃₋₇cycloalkylC₁₋₆alkyl, formyl, C₁₋₆alkylcarbonyl, carboxy, NR²R³, CONR²R³, S(O)ᵣNR²R³; or a ring which is phenyl; naphthyl; a five-membered heteroaromatic ring containing one, two, three or four heteroatoms independently chosen from O, N and S, at most one heteroatom being O or S; a six-membered heteroaromatic ring containing one, two or three N atoms; or a six-membered saturated ring containing one or two heteroatoms independently chosen from O and N; the ring being optionally substituted by one or more groups independently selected from halogen, C₁₋₆alkyl, C₂₋₆alkenyl, C₂₋₆alkynyl, nitro, cyano, C₃₋₇cycloalkyl, hydroxy, C₁₋₆alkoxy haloC₁₋₆alkyl, haloC₁₋₆alkoxy, hydroxyC₁₋₆alkyl, hydroxyC₁₋₆alkoxy and NR²R³;
R⁶ is hydrogen or C₁₋₆alkyl;
R⁷ and R⁸ are independently hydrogen, hydroxy, halogen or C₁₋₄alkyl;
Z is a phenyl ring, a five-membered heteroaromatic ring containing one, two, three or four heteroatoms independently chosen from O, N or S, at most one heteroatom being O or S, or a six-membered heteroaromatic ring containing one, two or three N atoms, optionally substituted by one or more groups chosen from halogen, hydroxy, cyano, nitro, NR²R³ or S(O)ᵣNR²R³ where NR²R³ is as defined above, C₁₋₆alkyl, C₂₋₆alkenyl, C₂₋₆alkynyl, haloC₁₋₆alkyl, C₁₋₆alkoxy, haloC₁₋₆alkoxy, C₁₋₆alkylthio, haloC₁₋₆alkylthio,C₃₋₇cycloalkyl, hydroxyC₁₋₃alkyl, a five-membered heteroaromatic ring containing 1, 2 or 3 heteroatoms independently chosen from O, N and S, providing that no more than one O or S atom is present, or a six-membered heteroaromatic ring containing 1, 2 or 3 N atoms;
a is zero, one, two, three or four,
p is zero, one, two or three;
r is one or two;
provided that when p is zero then R⁵ is not H; and when p is one or two and R⁵ is H then at least one group R¹ is other than halogen, hydroxy and C₁₋₆alkyl;
or a pharmaceutically acceptable salt or N-oxide thereof.

2. A compound according to claim 1 wherein:
w is 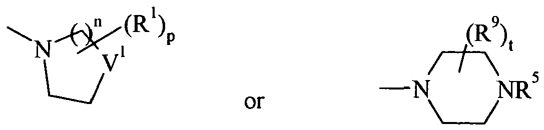 wherein R¹ is as defined above;
p is zero, one, two or three;
n is zero, one, two or three;
when n is zero or one, V¹ is CH₂;
when n is two or three, V¹ is CH₂ or O;
when V¹ is CH₂, the bond formed by V¹ and an adjacent carbon ring atom is optionally fused to a phenyl ring, a five membered heteroaromatic ring containing 1, 2 or 3 heteroatoms independently chosen from O, N and S, providing that no more than one O or S is present, or a six membered heteroaromatic ring containing 1, 2 or 3 N atoms; the ring being optionally substituted by one or more R¹ groups;
R⁵ is hydrogen or together with an adjacent N-C ring bond forms a fused five-membered heteroaromatic ring containing one, two, three or four nitrogen atoms optionally substituted by one or more R¹ groups;
when R⁵ is hydrogen, t is one, two or three;
when R⁵ together with an adjacent N-C ring bond forms a fused ring, t is zero, one, two or three; and
each R⁹ is independently cyano, haloC₁₋₆alkyl, hydroxyC₁₋₆alkyl, C₁₋₆alkoxy, haloC₁₋₆alkoxy, hydroxyC₁₋₆alkoxy, C₃₋₇cycloalkyl, C₃₋₇cycloalkylC₁₋₆alkyl, formyl, C₁₋₆alkylcarbonyl, carboxy, NR²R³, CONR²R³, S(O)ᵣNR²R³; or a ring which is phenyl; naphthyl; a five-membered heteroaromatic ring containing one, two, three or four heteroatoms independently chosen from O, N and S, at most one heteroatom being O or S; a six-membered heteroaromatic ring containing one, two or three N atoms; or a six-membered saturated ring containing one or two heteroatoms independently chosen from O and N, the ring being optionally substituted by one or more groups independently selected from halogen, C₁₋₆alkyl, C₂₋₆alkenyl, C₂₋₆alkynyl, nitro, cyano, C₃₋₇cycloalkyl, hydroxy, C₁₋₆alkoxy, haloC₁₋₆alkyl, haloC₁₋₆alkoxy, hydroxyC₁₋₆alkyl, hydroxyC₁₋₆alkoxy and NR²R³.

3. A compound according to claim 1 or 2 wherein V or V¹ is CH₂ or O and n is two or three.

4. A compound according to claim 1 or 2 wherein n is zero, one or two.

5. A compound according to any preceding claim wherein V or V¹ is CH₂ and a bond formed by V or V¹ to an adjacent carbon ring atom is fused to a ring selected from phenyl, pyridine, pyrimidine, thiophene or thiazole, the ring being optionally substituted by halogen or haloC₁₋₆alkyl.

6. A compound according to any preceding claim wherein Z is optionally substituted phenyl or pyridinyl.

7. A pharmaceutical composition comprising a compound of any one of claims 1 to 6 or a pharmaceutically acceptable salt or N-oxide thereof and a pharmaceutically acceptable carrier.

8. A compound of any one of claims 1 to 6 or a pharmaceutically acceptable salt or N-oxide thereof for use in a method of treatment of the human or animal body by therapy.

9. Use of a compound of any one of claims 1 to 6 or a pharmaceutically acceptable salt or N-oxide thereof for the manufacture of a medicament for the prevention or treatment of diseases and conditions in which pain and/or inflammation predominates, depression or gastro-oesophageal reflux disease.

10. Use according to claim 9 where the disease or condition is rheumatoid arthritis; osteoarthritis; post-surgical pain; musculo-skeletal pain, particularly after trauma; spinal pain; myofascial pain syndromes; headache, including migraine, acute or chronic tension headache, cluster headache, temporomandibular pain, and maxillary sinus pain; ear pain; episiotomy pain; bums, and especially primary hyperalgesia associated therewith; deep and visceral pain, such as heart pain, muscle pain, eye pain, orofacial pain, for example, odontalgia, abdominal pain, gynaecological pain, for example, dysmenorrhoea, pain associated with cystitis and labour pain, chronic pelvic pain, chronic prostatitis and endometriosis; pain associated with nerve and root damage, such as pain associated with peripheral nerve disorders, for example, nerve entrapment and brachial plexus avulsions, amputation, peripheral neuropathies, tic douloureux, atypical facial pain, nerve root damage, and arachnoiditis; itching conditions including pruritis, itch due to hemodialysis, and contact dermatitis; pain (as well as broncho-constriction and inflammation) due to exposure (e.g. via ingestion, inhalation, or eye contact) of mucous membranes to capsaicin and related irritants such as tear gas, hot peppers or pepper spray; neuropathic pain conditions such as diabetic neuropathy, chemotherapy-induced neuropathy and post-herpetic neuralgia; "non-painful" neuropathies; complex regional pain syndromes; pain associated with carcinoma, often referred to as cancer pain; central nervous system pain, such as pain due to spinal cord or brain stem damage, low back pain, sciatica and ankylosing spondylitis; gout; scar pain; irritable bowel syndrome; inflammatory bowel disease; urinary incontinence including bladder detrusor hyper-reflexia and bladder hypersensitivity; respiratory diseases including cough, chronic obstructive pulmonary disease (COPD), chronic bronchitis, cystic fibrosis, asthma and rhinitis, including allergic rhinitis such as seasonal and perennial rhinitis, and non-allergic rhinitis; autoimmune diseases; immunodeficiency disorders; and hot flushes.

## Patentansprüche

1. Eine Verbindung der Formel I: 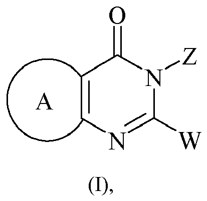 wobei:
W 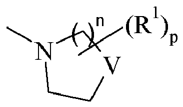 ist,
A ein fünfgliedriger heteroaromatischer Ring ist, der 2 N-Heteroatome enthält,
A gegebenenfalls substituiert ist mit einer, zwei oder drei Gruppen, unabhängig ausgewählt aus Halogen, Hydroxy, S(O)ᵣC₁₋₆-Alkyl, S(O)ᵣNR²R³, Formyl, C₁₋₆-Alkylcarbonyl, C₁₋₆-Alkyl, Halogen-C₁₋₆-alkyl, Hydroxy-C₁₋₆-alkyl, C₁₋₆-Alkoxy, Halogen-C₁₋₆-alkoxy, Hydroxy-C₁₋₆-alkoxy, C₃₋₇-Cycloalkyl, C₃₋₇-Cycloalkoxy, C₂₋₆-Alkenyl, C₂₋₆-Alkinyl, Amino, Nitro, Cyano, C₁₋₆-Alkylamino, Di(C₁₋₆-alkyl)amino, Amino-C₁₋₆-alkyl, Amino-C₁₋₆-alkoxy, C₁₋₆-Alkylamino-C₁₋₆-alkyl, Di(C₁₋₆-alkyl)amino-C₁₋₆-alkyl; und einem Ring, ausgewählt aus Phenyl, Naphthyl, einem fünfgliedrigen heteroaromatischen Ring, der ein, zwei, drei oder vier Heteroatome enthält, unabhängig ausgewählt aus O, N oder S, wobei höchstens ein Heteroatom O oder S ist, und einem sechsgliedrigen heteroaromatischen Ring, der ein, zwei oder drei N-Atome enthält, wobei der Ring gegebenenfalls substituiert ist durch Halogen, Hydroxy, Cyano, Nitro, NR²R³ wie nachstehend definiert, C₁₋₆-Alkyl, C₂₋₆-Alkenyl, C₂₋₆-Alkinyl, Halogen-C₁₋₆-alkyl, C₁₋₆-Alkoxy, Halogen-C₁₋₆-alkoxy, C₃₋₇-Cycloalkyl oder Hydroxy-C₁₋₆-alkyl,
R¹ X-Y-R⁴ ist,
jedes R² und R³ unabhängig Wasserstoff oder C₁₋₆-Alkyl ist, oder R² und R³ zusammen mit dem Stickstoffatom, an das sie gebunden sind, einen gesättigten 4-7-gliedrigen Ring bilden können,
n null, eins, zwei oder drei ist,
wenn n null oder eins ist, V CH₂ ist,
wenn n zwei oder drei ist, V CH₂, O oder NR⁵ ist,
wenn V CH₂ ist, die von V und einem benachbarten Kohlenstoffringatom gebildete Bindung gegebenenfalls an einen Phenylring, einen fünfgliedrigen heteroaromatischen Ring, der 1, 2 oder 3 Heteroatome enthält, unabhängig ausgewählt aus O, N und S, mit der Maßgabe, dass nicht mehr als ein O oder S vorliegt, oder einen sechsgliedrigen heteroaromatischen Ring, der 1, 2 oder 3 N-Atome enthält, kondensiert ist, wobei der Ring gegebenenfalls durch eine oder mehrere R¹-Gruppen substituiert ist,
R⁵ Wasserstoff ist oder zusammen mit einer benachbarten N-C-Ring-Bindung einen kondensierten fünfgliedrigen heteroaromatischen Ring bildet, der ein, zwei, drei oder vier Stickstoffatome enthält und gegebenenfalls durch eine oder mehrere R¹-Gruppen substituiert ist,
X eine Bindung, O oder NR⁶ ist,
Y (CR⁷R⁸)ₐ ist,
jedes R⁴ unabhängig Halogen, Hydroxy, Cyano, C₁₋₆-Alkyl, Halogen-C₁₋₆-alkyl, Hydroxy-C₁₋₆-alkyl, C₁₋₆-Alkoxy, Halogen-C₁₋₆-alkoxy, Hydroxy-C₁₋₆-alkoxy, C₃₋₇-Cycloalkyl, C₃₋₇-Cycloalkyl-C₁₋₆-alkyl, Formyl, C₁₋₆-Alkylcarbonyl, Carboxy, NR²R³, CONR²R³, S(O)ᵣNR²R³ ist oder ein Ring, der Phenyl, Naphthyl, ein fünfgliedriger heteroaromatischer Ring, der ein, zwei, drei oder vier Heteroatome, unabhängig ausgewählt aus O, N und S, enthält, wobei höchstens ein Heteroatom O oder S ist, ein sechsgliedriger heteroaromatischer Ring, der ein, zwei oder drei N-Atome enthält, oder ein sechsgliedriger gesättigter Ring, der ein oder zwei Heteroatome enthält, unabhängig ausgewählt aus O und N, ist, wobei der Ring gegebenenfalls substituiert ist durch eine oder mehrere Gruppen, unabhängig ausgewählt aus Halogen, C₁₋₆-Alkyl, C₂₋₆-Alkenyl, C₂₋₆-Alkinyl, Nitro, Cyano, C₃₋₇-Cycloalkyl, Hydroxy, C₁₋₆-Alkoxy, Halogen-C₁₋₆-alkyl, Halogen-C₁₋₆-alkoxy, Hydroxy-C₁₋₆-alkyl, Hydroxy-C₁₋₆-alkoxy und NR²R³,
R⁶ Wasserstoff oder C₁₋₆-Alkyl ist,
R⁷ und R⁸ unabhängig Wasserstoff, Hydroxy, Halogen oder C₁₋₄-Alkyl sind,
Z ein Phenylring, ein fünfgliedriger heteroaromatischer Ring, der ein, zwei, drei oder vier Heteroatome enthält, unabhängig ausgewählt aus O, N oder S, wobei höchstens ein Heteroatom O oder S ist, oder ein sechsgliedriger heteroaromatischer Ring, der ein, zwei oder drei N-Atome enthält, ist, gegebenenfalls substituiert durch eine oder mehrere Gruppen, ausgewählt aus Halogen, Hydroxy, Cyano, Nitro, NR²R³ oder S(O)ᵣNR²R³, wobei NR²R³ wie oben definiert ist, C₁₋₆-Alkyl, C₂₋₆-Alkenyl, C₂₋₆-Alkinyl, Halogen-C₁₋₆-alkyl, C₁₋₆-Alkoxy, Halogen-C₁₋₆-alkoxy, C₁₋₆-Alkylthio, Halogen-C₁₋₆-alkylthio, C₃₋₇-Cycloalkyl, Hydroxy-C₁₋₆-alkyl, einem fünfgliedrigen heteroaromatischen Ring, der 1, 2 oder 3 Heteroatome enthält, unabhängig ausgewählt aus O, N und S, mit der Maßgabe, dass nicht mehr als ein O- oder S-Atom vorliegt, oder einem sechsgliedrigen heteroaromatischen Ring, der 1, 2 oder 3 N-Atome enthält,
a null, eins, zwei, drei oder vier ist,
p null, eins, zwei oder drei ist,
r eins oder zwei ist,
mit der Maßgabe, dass, wenn p null ist, R⁵ nicht H ist, und dass wenn p eins oder zwei ist und R⁵ H ist, wenigstens eine Gruppe R¹ anders als Halogen, Hydroxy und C₁₋₆-Alkyl ist,
oder ein pharmazeutisch annehmbares Salz oder N-Oxid davon.

2. Eine Verbindung gemäß Anspruch 1, wobei:
W 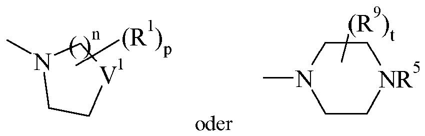 ist, wobei R¹ wie oben definiert ist,
p null, eins, zwei oder drei ist,
n null, eins, zwei oder drei ist,
wenn n null oder eins ist, V¹ CH₂ ist,
wenn n zwei oder drei ist, V¹ CH₂ oder O ist,
wenn V¹ CH₂ ist, die von V¹ und einem benachbarten Kohlenstoffringatom gebildete Bindung gegebenenfalls an einen Phenylring, einen fünfgliedrigen heteroaromatischen Ring, der 1, 2 oder 3 Heteroatomen enthält, unabhängig ausgewählt aus O, N und S, mit der Maßgabe, dass nicht mehr als ein O oder S vorliegt, oder einen sechsgliedrigen heteroaromatischen Ring, der 1, 2 oder 3 N-Atome enthält, kondensiert ist, wobei der Ring gegebenenfalls durch eine oder mehrere R¹-Gruppen substituiert ist,
R⁵ Wasserstoff ist oder zusammen mit einer benachbarten N-C-Ring-Bindung einen kondensierten fünfgliedrigen heteroaromatischen Ring bildet, der ein, zwei, drei oder vier Stickstoffatome enthält und gegebenenfalls durch eine oder mehrere R¹-Gruppen substituiert ist,
wenn R⁵ Wasserstoff ist, t eins, zwei oder drei ist,
wenn R⁵ zusammen mit einer benachbarten N-C-Ring-Bindung einen kondensierten Ring bildet, t null, eins, zwei oder drei ist und
jedes R⁹ unabhängig Cyano, Halogen-C₁₋₆-alkyl, Hydroxy-C₁₋₆-alkyl, C₁₋₆-Alkoxy, Halogen-C₁₋₆-alkoxy, Hydroxy-C₁₋₆-alkoxy, C₃₋₇-Cycloalkyl, C₃₋₇-Cycloalkyl-C₁₋₆-alkyl, Formyl, C₁₋₆-Alkylcarbonyl, Carboxy, NR²R³, CONR²R³, S(O)ᵣNR²R³ oder ein Ring ist, wobei der Ring Phenyl, Naphthyl, ein fünfgliedriger heteroaromatischer Ring, der ein, zwei, drei oder vier Heteroatome, unabhängig ausgewählt aus O, N und S, enthält, wobei höchstens ein Heteroatom O oder S ist, ein sechsgliedriger heteroaromatischer Ring, der ein, zwei oder drei N-Atome enthält, oder ein sechsgliedriger gesättigter Ring, der ein oder zwei Heteroatome enthält, unabhängig ausgewählt aus O und N, ist, wobei der Ring gegebenenfalls substituiert ist mit einer oder mehreren Gruppen, unabhängig ausgewählt aus Halogen, C₁₋₆-Alkyl, C₂₋₆-Alkenyl, C₂₋₆-Alkinyl, Nitro, Cyano, C₃₋₇-Cycloalkyl, Hydroxy, C₁₋₆-Alkoxy, Halogen-C₁₋₆-alkyl, Halogen-C₁₋₆-alkoxy, Hydroxy-C₁₋₆-alkyl, Hydroxy-C₁₋₆-alkoxy und NR²R³.

3. Eine Verbindung gemäß Anspruch 1 oder 2, wobei V oder V¹ CH₂ oder O ist und n zwei oder drei ist.

4. Eine Verbindung gemäß Anspruch 1 oder 2, wobei n null, eins oder zwei ist.

5. Eine Verbindung gemäß einem vorhergehenden Anspruch, wobei V und V¹ CH₂ ist und eine Bindung, die von V oder V¹ an ein benachbartes Kohlenstoffringatom gebildet wird, an einen Ring kondensiert ist, ausgewählt aus Phenyl, Pyridin, Pyrimidin, Thiophen oder Thiazol, wobei der Ring gegebenenfalls substituiert ist durch Halogen oder Halogen-C₁₋₆-alkyl.

6. Eine Verbindung gemäß einem vorhergehenden Anspruch, wobei Z gegebenenfalls substituiertes Phenyl oder Pyridinyl ist.

7. Eine pharmazeutische Zusammensetzung, die eine Verbindung nach einem der Ansprüche 1 bis 6 oder ein pharmazeutisch annehmbares Salz oder N-Oxid davon und einen pharmazeutisch annehmbaren Träger umfasst.

8. Eine Verbindung nach einem der Ansprüche 1 bis 6 oder ein pharmazeutisch annehmbares Salz oder N-Oxid davon zur Verwendung bei einem Verfahren zur Behandlung des Körpers eines Menschen oder eines Tiers durch Therapie.

9. Verwendung einer Verbindung nach einem der Ansprüche 1 bis 6 oder eines pharmazeutisch annehmbaren Salzes oder N-Oxids davon zur Herstellung eines Medikaments zur Prävention oder Behandlung von Erkrankungen und Zuständen, bei denen Schmerz und/oder Entzündung vorherrscht, Depression oder Refluxösophagitis.

10. Verwendung gemäß Anspruch 9, wobei die Erkrankung oder der Zustand rheumatoide Arthritis; Osteoarthritis; postoperative Schmerzen; Muskuloskeletalschmerzen, speziell nach einem Trauma; Rückenschmerzen; myofasziale Schmerzsyndrome; Kopfschmerzen, einschließlich Migräne, akuten oder chronischen Spannungskopfschmerzen, Cluster-Kopfschmerzen, Myoarthropathie und Kieferhöhlenschmerzen; Ohrenschmerzen; Episiotomie-Schmerzen; Verbrennungen und speziell damit verbundene primäre Hyperalgesie; tiefe und viszerale Schmerzen, wie z.B. Herzschmerzen, Muskelschmerzen, Augenschmerzen, orofazialen Schmerzen, zum Beispiel Zahnschmerzen, Bauchschmerzen, gynäkologischen Schmerzen, zum Beispiel Dysmenorrhöen, Schmerzen in Verbindung mit Zystitis und Wehenschmerzen, chronischen Beckenschmerzen, chronische Prostatitis und Endometriose; Schmerzen in Verbindung mit Nerven- und Wurzelschädigung, wie z.B. Schmerzen in Verbindung mit peripheren Nervenstörungen, zum Beispiel Nerveneinklemmung und Plexus-Brachialis-Rissen, Amputation, periphere Neuropathien, Tic douloureux, atypische Gesichtsschmerzen, Nervenwurzelschädigung und Arachnoiditis; Juckzustände, einschließlich Pruritis, Juckreiz durch Hämodialyse, und Kontaktdermatitis; Schmerzen (sowie Bronchokonstriktion und Entzündung) aufgrund des Kontakts (z.B. durch Einnahme, Inhalation oder Augenkontakt) von Schleimhäuten mit Capsaicin und verwandten Reizstoffen, wie z.B. Tränengas, scharfen Paprikaschoten oder Pfefferspray; neuropathische Schmerzzustände, wie z.B. diabetische Neuropathie, chemotherapieinduzierte Neuropathie und postherpetische Neuralgie; "nicht schmerzhafte" Neuropathien; komplexe örtliche Schmerzsyndrome; Schmerzen in Verbindung mit Karzinomen, die oftmals als Krebsschmerzen bezeichnet werden; Zentralnervensystemschmerzen, wie z.B. Schmerzen aufgrund von Schädigung des Rückenmarks oder Hirnstamms, Schmerzen des unteren Rückens, Ischiassyndrom und Spondylitis ankylosans; Gicht; Narbenschmerzen; Reizdarmsyndrom; entzündliche Darmerkrankung; Harninkontinenz, einschließlich Blasendetrusor-Hyperreflexie und Blasenüberempfindlichkeit; Atemwegserkrankungen, einschließlich Husten, chronischer obstruktiver Lungenerkrankung (COPD), chronischer Bronchitis, zystischer Fibrose, Asthma und Rhinitis, einschließlich allergischer Rhinitis, wie z.B. jahreszeitlich bedingte und dauernde Rhinitis, und nichtallergischer Rhinitis; Autoimmunerkrankungen; Immundefektstörungen und Hitzewallungen ist.

## Revendications

1. Composé de la formule I: 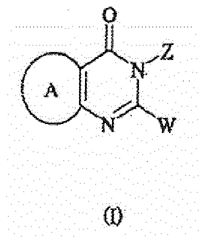 dans lequel:
W est 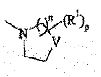
A est un cycle hétéroaromatique à 5 chaînons contenant 2 hétéroatomes N;
A est optionnellement substitué par un, deux ou trois groupes sélectionnés indépendamment parmi halogène, hydroxy, S(O)ᵣalkyle C₁₋₆, S(O)ᵣNR²R³, formyle, alkylcarbonyle C₁₋₆, alkyle C₁₋₆, haloalkyle C₁₋₆, hydroxyalkyle C₁₋₆, alcoxy C₁₋₆, haloalcoxy C₁₋₆, hydroxyalcoxy C₁₋₆, cycloalkyle C₃₋₇, cycloalcoxy C₃₋₇, alcényle C₂₋₆, alcynyle C₂₋₆, amino, nitro, cyano, alkylamino C₁₋₆, di(alkyle C₁₋₆)amino, amino alkyle C₁₋₆, aminoalcoxy C₁₋₆, alkyle C₁₋₆-aminoalkyle C₁₋₆, di(alkyle C₁₋₆)aminoalkyle C₁₋₆; et un cycle sélectionné parmi phényle, naphtyle, un cycle hétéroaromatique à 5 chaînons contenant un, deux, trois ou quatre hétéroatomes sélectionnés indépendamment parmi O, N ou S, un hétéroatome au plus étant O ou S et un cycle hétéroaromatique à 6 chaînons contenant un, deux ou trois atomes N, le cycle étant optionnellement substitué par halogène, hydroxy, cyano, nitro, NR²R³ comme il est défini ci-dessous, alkyle C₁₋₆, alcényle C₂₋₆, alcynyle C₂₋₆, haloalkyle C₁₋₆, alcoxy C₁₋₆, haloalcoxy C₁₋₆, cycloalkyle C₃₋₇ ou hydroxyalkyle C₁₋₆;
R¹ est X-Y-R⁴,
chaque R² et R³ est indépendamment hydrogène ou alkyle C₁₋₆ ou bien R² et R³, ensemble avec l'atome d'azote auquel ils sont attachés, peuvent former un cycle saturé à 4-7 chaînons;
n est zéro, un, deux ou trois;
lorsque n est zéro ou un, V est CH₂;
lorsque n est deux ou trois, V est CH₂, O ou NR⁵;
lorsque V est CH₂, la liaison formée par V et un atome de carbone cyclique adjacent est optionnellement fusionnée à un cycle phényle, un cycle hétéroaromatique à cinq chaînons contenant 1, 2 ou 3 hétéroatomes sélectionnés indépendamment parmi O, N et S, à condition que pas plus d'un O ou S ne soit présent, ou un cycle hétéroaromatique à six chaînons contenant 1, 2 ou 3 atomes N; le cycle étant optionnellement substitué par un ou plusieurs groupes R¹;
R⁵ est hydrogène ou bien ensemble avec une liaison cyclique N-C adjacente, forme un cycle hétéroaromatique condensé à cinq chaînons contenant un, deux, trois ou quatre atomes d'azote, optionnellement substitué par un ou plusieurs groupes R¹;
X est une liaison, O ou NR⁶,
Y est (CR⁷R⁸)ₐ;
chaque R⁴ est indépendamment halogène, hydroxy, cyano, alkyle C₁₋₆, haloalkyle C₁₋₆, hydroxyalkyle C₁₋₆, alcoxy C₁₋₆, haloalcoxy C₁₋₆, hydroxyalcoxy C₁₋₆, cycloalkyle C₃₋₇, cycloalkyle C₃₋₇-alkyle C₁₋₆, formyle, alkylcarbonyle C₁₋₆, carboxy, NR²R³, CONR²R³, S(O)ᵣNR²R³; ou un cycle qui est phényle; naphtyle; un cycle hétéroaromatique à cinq chaînons contenant un, deux, trois ou quatre hétéroatomes sélectionnés indépendamment parmi O, N et S, un hétéroatome au plus étant O ou S; un cycle hétéroaromatique à six chaînons contenant un, deux ou trois atomes N; ou un cycle saturé à six chaînons contenant un ou deux hétéroatomes sélectionnés indépendamment parmi O et N; le cycle étant optionnellement substitué par un ou plusieurs groupes sélectionnés indépendamment parmi halogène, alkyle C₁₋₆, alcényle C₂₋₆, alcynyle C₂₋₆, nitro, cyano, cycloalkyle C₃₋₇, hydroxy, alcoxy C₁₋₆, haloalkyle C₁₋₆, haloalcoxy C₁₋₆, hydroxyalkyle ₁₋₆, hydroxyalcoxy C₁₋₆ et NR²R³;
R⁶ est hydrogène ou alkyle C₁₋₆;
R⁷ et R⁸ sont indépendamment hydrogène, hydroxy, halogène ou alkyle C₁₋₄;
Z est un cycle phényle, un cycle hétéroaromatique à cinq chaînons contenant un, deux, trois ou quatre hétéroatomes sélectionnés indépendamment parmi O, N ou S, un hétéroatome au plus étant O ou S, ou un cycle hétéroaromatique à six chaînons contenant un, deux ou trois atomes N, optionnellement substitué par un ou plusieurs groupes sélectionnés parmi halogène, hydroxy, cyano, nitro, NR²R³ ou S(O)ᵣNR²R³ où NR²R³ est tel que défini ci-dessus, alkyle C₁₋₆, alcényle C₂₋₆, alcynyle C₂₋₆, haloalkyle C₁₋₆, alcoxy C₁₋₆, haloalcoxy C₁₋₆, alkylthio C₁₋₆, haloalkylthio C₁₋₆, cycloalkyle C₃₋₇, hydroxyalkyle C₁₋₆, un cycle hétéroaromatique à cinq chaînons contenant 1, 2 ou 3 hétéroatomes sélectionnés indépendamment parmi O, N et S, à condition que pas plus d'un atome O ou S ne soit présent, ou un cycle hétéroaromatique à six chaînons contenant 1, 2 ou 3 atomes N;
a est zéro, un, deux, trois ou quatre;
p est zéro, un, deux ou trois;
r est un ou deux;
à condition que lorsque p est zéro alors R⁵ n'est pas H; et lorsque p est un ou deux et R⁵ est H, alors au moins un groupe R¹ est autre qu'halogène, hydroxy et alkyle C₁₋₆;
ou un sel ou un N-oxyde pharmaceutiquement acceptable de celui-ci.

2. Composé selon la revendication 1, dans lequel:
W est 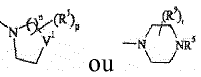 dans lequel R¹ est tel que défini ci-dessus;
p est zéro, un, deux ou trois;
n est zéro, un, deux ou trois;
lorsque n est zéro ou un, V¹ est CH₂;
lorsque n est deux ou trois, V¹ est CH₂ ou O;
lorsque V¹ est CH₂, la liaison formée par V¹ et un atome de carbone cyclique adjacent est optionnellement fusionnée à un cycle phényle, un cycle hétéroaromatique à cinq chaînons contenant 1, 2 ou 3 hétéroatomes sélectionnés indépendamment parmi O, N et S, à condition que pas plus d'un O ou S ne soit présent, ou un cycle hétéroaromatique à six chaînons contenant 1, 2 ou 3 atomes N; le cycle étant optionnellement substitué par un ou plusieurs groupes R¹;
R⁵ est hydrogène ou ensemble avec une liaison cyclique N-C adjacente, forme un cycle hétéroaromatique condensé à cinq chaînons contenant un, deux, trois ou quatre atomes d'azote, optionnellement substitué par un ou plusieurs groupes R¹;
lorsque R⁵ est hydrogène, t est un, deux ou trois;
lorsque R⁵ ensemble avec une liaison cyclique N-C adjacente, forme un cycle condensé, t est zéro, un, deux ou trois; et
chaque R⁹ est indépendamment cyano, haloalkyle C₁₋₆, hydroxyalkyle C₁₋₆, alcoxy C₁₋₆, haloalcoxy C₁₋₆, hydroxyalcoxy C₁₋₆, cycloalkyle C₃₋₇, cycloalkyle C₃₋₇-alkyle C₁₋₆, formyle, alkylcarbonyle C₁₋₆, carboxy, NR²R³, CONR²R³, S(O)ᵣNR²R³; ou un cycle qui est phényle; naphtyle; un cycle hétéroaromatique à cinq chaînons contenant un, deux, trois ou quatre hétéroatomes sélectionnés indépendamment parmi O, N et S, un hétéroatome au plus étant O ou S; un cycle hétéroaromatique à six chaînons contenant un, deux ou trois atomes N; ou un cycle saturé à six chaînons contenant un ou deux hétéroatomes sélectionnés indépendamment parmi O et N, le cycle étant optionnellement substitué par un ou plusieurs groupes sélectionnés indépendamment parmi halogène, alkyle C₁₋₆, alcényle C₂₋₆, alcynyle C₂₋₆, nitro, cyano, cycloalkyle C₃₋₇, hydroxy, alcoxy C₁₋₆, haloalkyle C₁₋₆, haloalcoxy C₁₋₆, hydroxyalkyle C₁₋₆, hydroxyalcoxy C₁₋₆ et NR²R³.

3. Composé selon la revendication 1 ou 2, dans lequel V ou V¹ est CH₂ ou O et n est deux ou trois.

4. Composé selon la revendication 1 ou 2, dans lequel n est zéro, un ou deux.

5. Composé selon l'une quelconque des revendications précédentes, dans lequel V ou V¹ est CH₂ et une liaison formée par V ou V¹ avec un atome de carbone cyclique adjacent est fusionnée à un cycle sélectionné parmi phényle, pyridine, pyrimidine, thiophène ou thiazole, le cycle étant optionnellement substitué par halogène ou haloalkyle C₁₋₆.

6. Composé selon l'une quelconque des revendications précédentes, dans lequel Z est optionnellement substitué par phényle ou pyridinyle.

7. Composition pharmaceutique comprenant un composé selon l'une quelconque des revendications 1 à 6 ou un sel ou N-oxyde pharmaceutiquement acceptable de celui-ci et un véhicule pharmaceutiquement acceptable.

8. Composé selon l'une quelconque des revendications 1 à 6 ou un sel ou un N-oxyde pharmaceutiquement acceptable de celui-ci, à utiliser dans une méthode de traitement du corps humain ou animal par thérapie.

9. Utilisation d'un composé selon l'une quelconque des revendications 1 à 6 ou d'un sel ou N-oxyde pharmaceutiquement acceptable de celui-ci, dans la fabrication d'un médicament pour la prévention ou le traitement de maladies et de conditions dans lesquelles la douleur et/ou l'inflammation prédominent, la dépression ou une maladie de reflux gastro-oesophagien.

10. Utilisation selon la revendication 9, dans laquelle la maladie ou la condition est la polyarthrite rhumatoïde; l'ostéoarthrite; la douleur post-opératoire; la douleur musculosquelettique, surtout suite à un traumatisme; la douleur rachidienne; les syndromes de douleur myofasciale; la céphalée, y compris migraine, céphalée aiguë ou chronique dite de tension, céphalée en grappe, douleur de l'articulation temporomandibulaire et douleur du sinus maxillaire; mal aux oreilles; douleur suite à une épisiotomie; brûlures et particulièrement hyperalgésie primaire associée à celles-ci; douleur profonde et viscérale, telle que douleur cardiaque, douleur musculaire, douleur oculaire, douleur oro-faciale, par exemple, odontalgie, douleur abdominale, douleur gynécologique, par exemple, dysménorrhée, douleur associée à la cystite et douleur d'accouchement, douleur pelvienne chronique, prostatite et endométriose chroniques; douleur associée à une lésion des nerfs et racines nerveuses, telle que douleur associée à des troubles des nerfs périphériques, par exemple nerf coincé et avulsions du plexus brachial, amputation, neuropathies périphériques, tic douloureux, douleur faciale atypique, lésion des racines nerveuses et arachnoïdite; conditions de démangeaison y compris prurit, démangeaison due à l'hémodialyse et dermatite de contact; douleur (ainsi que bronchoconstriction et inflammation) par exposition (par ex. par ingestion, inhalation ou contact oculaire) des membranes muqueuses à la capsaïcine et irritants apparentés tels que gaz lacrymogène, piments forts ou gaz poivré; conditions de douleur neuropathique telle que neuropathie diabétique, neuropathie induite par la chimiothérapie et névralgie post-herpétique; neuropathies "non douloureuses"; syndromes douloureux régionaux complexes; douleur associée au carcinome, souvent appelée douleur cancéreuse; douleur du système nerveux central, telle douleur causée par une lésion rachidienne ou du tronc cérébral, douleur lombaire, sciatique et spondylite ankylosante; goutte; douleur cicatricielle; syndrome de l'intestin irritable; maladie inflammatoire de l'intestin; incontinence urinaire y compris hyper-réflexie du détrusor vésical et hypersensibilité vésicale; maladie respiratoire y compris toux, maladie pulmonaire obstructive chronique (MPOC), bronchite chronique, fibrose kystique, asthme et rhinite, y compris rhinite allergique telle que rhinite saisonnière et persistante et rhinite non allergique; maladies auto-immunes; troubles d'immunodéficience et bouffées de chaleur.
